# EUROPEAN PATENT SPECIFICATION

(11) **EP 1 809 620 B1**
(45) Date of publication and mention of the grant of the patent: **29.12.2010**
(21) Application number: 05801781.5
(22) Date of filing: 02.11.2005
(51) Int. Cl.: C07D 401/04, C07D 413/14, C07D 401/14, A61K 31/454, A61K 31/4545, A61P 25/18, A61P 25/28

(54) **NOVEL TETRAZOLE DERIVATIVES AS POSITIVE ALLOSTERIC MODULATORS OF METABOTROPIC GLUTAMATE RECEPTORS**
NEUARTIGE TETRAZOLDERIVATE ALS POSITIVE ALLOSTERISCHE MODULATOREN VON METABOTROPEN GLUTAMATREZEPTOREN
NOUVEAUX DERIVES DE TETRAZOLE UTILISES COMME MODULATEURS ALLOSTERIQUES POSITIFS DES RECEPTEURS DE GLUTAMATE METABOTROPIQUES

(30) Priority: 04.11.2004 WO PCT/IB2004/003822; 18.05.2005 GB 0510143
(43) Date of publication of application: 25.07.2007
(73) Proprietor: Addex Pharma SA, 1228 Plan-les-Ouates (CH)
(72) Inventor: GAGLIARDI, Stefania, 20021 Baranzate di Bollate (IT); PALOMBI, Giovanni, 20021 Baranzate di Bollate (IT); ROCHER, Jean-Philippe, 1228 Plan les Ouates (CH)
(74) Representative: Davies, Jonathan Mark
(86) International application number: PCT/IB2005/003498
(87) International publication number: WO 2006/048771

(56) References cited:
- WO-A-2003/029210
- WO-A-2003/077918
- WO-A-2004/044797
- WO-A-2005/080386
- US-A1- 2004 186 295
- D HUANG ET. AL.: "2-[2-[3-(Pyridin-3-yloxy)phenyl]-2H-tetra zol-5-yl}pyridine. A highly piotent, orally active, metabotropic glutamate subtype 5 (MGluR5) receptor antagonist." BIOORGANIC AND MEDICINAL CHEMISTRY LETTERS, vol. 14, 2004, pages 5473-5476, XP004598576
- A.B. PINKERTON ET. AL.: "Allosteric potentiators of the metabotropic glutamate receptor 2 (mGluR2). Part 1. Identification and synthesis of phenyl tetrazolyl acetophenones." BIOORGANIC AND MEDICINAL CHEMISTRY LETTERS, vol. 14, 2004, pages 5329-5332, XP004580524

## Description

### FIELD OF THE INVENTION

The present invention provides new tetrazole compounds of formula I as defined in claim 1 as positive allosteric modulators of metabotropic receptors - subtype 5 ("mGluR5") which are useful for the treatment or prevention of central nervous system disorders such as for example: cognitive decline, both positive and negative symptoms in schizophrenia as well as other disorders in which the mGluR5 subtype of glutamate metabotropic receptor is involved. The invention is also directed to use of compounds and pharmaceutical compositions in the prevention or treatment of such diseases in which mGluR5 is involved.

### BACKGROUND OF THE INVENTION

Glutamate, the major amino-acid transmitter in the mammalian central nervous system (CNS), mediates excitatory synaptic neurotransmission through the activation of ionotropic glutamate receptors receptor-channels (iGluRs, namely NMDA, AMPA and kainate) and metabotropic glutamate receptors (mGluRs). iGluRs are responsible for fast excitatory transmission (Nakanishi S et al., (1998) Brain Res. Rev., 26:230-235) while mGluRs have a more modulatory role that contributes to the fine-tuning of synaptic efficacy. Glutamate performs numerous physiological functions such as long-term potentiation (LTP), a process believed to underlie learning and memory but also cardiovascular regulation, sensory perception, and the development of synaptic plasticity. In addition, glutamate plays an important role in the patho-physiology of different neurological and psychiatric diseases, especially when an imbalance in glutamatergic neurotransmission occurs.

The mGluRs are seven-transmembrane G protein-coupled receptors. The eight members of the family are classified into three groups (Groups I, II & III) according to their sequence homology and pharmacological properties (Schoepp DD et al. (1999) Neuropharmacology, 38:1431-1476). Activation of mGluRs lead to a large variety of intracellular responses and activation of different transductional cascades. Among mGluR members, the mGluR5 subtype is of high interest for counterbalancing the deficit or excesses of neurotransmission in neuropsychatric diseases. mGluR5 belongs to Group I and its activation initiates cellular responses through G-protein mediated mechanisms. mGluR5 is coupled to phospholipase C and stimulates phosphoinositide hydrolysis and intracellular calcium mobilization. mGluR5 proteins have been demonstrated to be localized in post-synaptic elements adjacent to the post-synaptic density (Lujan R et al. (1996) Eur. J. Neurosci., 8:1488-500; Lujan R et al. (1997) J. Chem. Neuroanat., 13:219-41) and are rarely detected in the pre-synaptic elements (Romano C et al. (1995) J. Comp. Neurol., 355:455-69). mGluR5 receptors can therefore modify the post-synaptic responses to neurotransmitter or regulate neurotransmitter release.

In the CNS, mGluR5 receptors are abundant mainly throughout the cortex, hippocampus, caudate-putamen and nucleus accumbens. As these brain areas have been shown to be involved in emotion, motivational processes and in numerous aspects of cognitive function, mGluR5 modulators are predicted to be of therapeutic interest.

A variety of potential clinical indications have been suggested to be targets for the development of subtype selective mGluR modulators. These include epilepsy, neuropathic and inflammatory pain, numerous psychiatric disorders (eg anxiety and schizophrenia), movement disorders (eg Parkinson disease), neuroprotection (stroke and head injury), migraine and addiction/drug dependency (for reviews, see Brauner-Osbome H et al. (2000) J. Med. Chem., 43:2609-45; Bordi F and Ugolini A. (1999) Prog. Neurobiol., 59:55-79; Spooren W et al. (2003) Behav. Pharmacol., 14:257-77).

The hypothesis of hypofunction of the glutamatergic system as reflected by NMDA receptor hypofunction as a putative cause of schizophrenia has received increasing support over the past few years (Goff DC and Coyle JT (2001) Am. J. Psychiatry, 158:1367-1377; Carlsson A et al. (2001) Annu. Rev. Pharmacol. Toxicol., 41:237-260 for a review). Evidence implicating dysfunction of glutamatergic neurotransmission is supported by the finding that antagonists of the NMDA subtype of glutamate receptor can reproduce the full range of symptoms as well as the physiologic manifestation of schizophrenia such as hypofrontality, impaired prepulse inhibition and enhanced subcortical dopamine release. In addition, clinical studies have suggested that mGluR5 allele frequency is associated with schizophrenia among certain cohorts (Devon RS et al. (2001) Mol. Psychiatry., 6:311-4) and that an increase in mGluR5 message has been found in cortical pyramidal cells layers of schizophrenic brain (Ohnuma T et al. (1998) Brain Res. Mol. Brain Res., 56:207-17).

The involvement of mGluR5 in neurological and psychiatric disorders is supported by evidence showing that in vivo activation of group I mGluRs induces a potentiation of NMDA receptor function in a variety of brain regions mainly through the activation of mGluR5 receptors (Mannaioni G et al. (2001) Neurosci., 21:5925-34; Awad H et al. (2000) J. Neurosci., 20:7871-7879; Pisani A et al. (2001) Neuroscience, 106:579-87; Benquet P et al (2002) J. Neurosci., 22:9679-86).

The role of glutamate in memory processes also has been firmly established during the past decade (Martin SJ et al. (2000) Annu. Rev. Neurosci., 23:649-711; Baudry M and Lynch G. (2001) Neurobiol. Learn. Mem., 76:284-297). The use of mGluR5 null mutant mice have strongly supported a role of mGluR5 in learning and memory. These mice show a selective loss in two tasks of spatial learning and memory, and reduced CA1 LTP (Lu et al. (1997) J. Neurosci., 17:5196-5205; Schulz B et al. (2001) Neuropharmacology, 41:1-7; Jia Z et al. (2001) Physiol. Behav., 73:793-802; Rodrigues et al. (2002) J. Neurosci., 22:5219-5229).

The finding that mGluR5 is responsible for the potentiation of NMDA receptor mediated currents raises the possibility that agonists of this receptor could be useful as cognitive-enhancing agents, but also as novel antipsychotic agents that act by selectively enhancing NMDA receptor function.

The activation of NMDARs could potentiate hypofunctional NMDARs in neuronal circuitry relevant to schizophrenia. Recent in vivo data strongly suggest that mGluR5 activation may be a novel and efficacious approach to treat cognitive decline and both positive and negative symptoms in schizophrenia (Kinney GG et al. (2003) J. Pharmacol. Exp. Ther., 306(1):116-123).

mGluR5 receptor is therefore being considered as a potential drug target for treatment of psychiatric and neurological disorders including treatable diseases in this connection are anxiety disorders, attentional disorders, eating disorders, mood disorders, psychotic disorders, cognitive disorders, personality disorders and substance-related disorders.

Most of the current modulators of mGluR5 function have been developed as structural analogues of glutamate, quisqualate or phenylglycine (Schoepp DD et al. (1999) Neuropharmacology, 38:1431-1476) and it has been very challenging to develop in vivo active and selective mGluR5 modulators acting at the glutamate binding site. A new avenue for developing selective modulators is to identify molecules that act through allosteric mechanisms, modulating the receptor by binding to site different from the highly conserved orthosteric binding site.

Positive allosteric modulators of mGluRs have emerged recently as novel pharmacological entities offering this attractive alternative. This type of molecule has been discovered for mGluRl, mGluR2, mGluR4, and mGluR5 (Knoflach F et al. (2001) Proc. Natl. Acad. Sci. U S A., 98:13402-13407; O'Brien JA et al. (2003) Mol. Pharmacol., 64:731-40; Johnson K et al. (2002) Neuropharmacology, 43:291; Johnson MP et al. (2003) J. Med. Chem., 46:3189-92; Marino MJ et al. (2003) Proc. Natl. Acad. Sci. U S A., 100(23): 13668-73; for a review see Mutel V (2002) Expert Opin. Ther. Patents, 12:1-8; Kew JN (2004) Pharmacol. Ther., 104(3):233-44; Johnson MP et al. (2004) Biochem. Soc. Trans., 32:881-7). DFB and related molecules were described as in vitro mGluR5 positive allosteric modulators but with low potency (O'Brien JA et al. (2003) Mol. Pharmacol., 64:731-40). Benzamide derivatives have been patented (WO 2004/087048; O'Brien JA (2004) J. Pharmacol. Exp. Ther., 309:568-77) and recently aminopyrazole derivatives have been disclosed as mGluR5 positive allosteric modulators (Lindsley et al. (2004) J. Med. Chem., 47:5825-8; WO 2005/087048). Among aminopyrazole derivatives, CDPPB has shown in vivo activity antipsychotic-like effects in rat behavioral models (Kinney GG et al. (2005) J. Pharmacol. Exp. Ther., 313:199-206). This report is consistent with the hypothesis that allosteric potentiation of mGluR5 may provide a novel approach for development of antipsychotic agents.

None of the specifically disclosed compounds are structurally related to the compounds of the present invention.

Document (A) WO-A-2003/077918 describes modulators of the metabotropic glutamate receptor 5 (mGluR5) based on tetrazoles substituted at the 2 and 5 positions by aryl or heteroaryl groups. The aryl or heteroaryl groups may also be further substituted by aryl groups which are directly bound or linked via a CH₂, O or NH group. The presently claimed compounds differ from those of (A) in that the 5-position of the tetrazole moiety is substituted by a piperidine group. Documents (B) WO-A-2003/029210 and (C) USA-2004/186295 also describe further mGluR5 modulators based on tetrazoles substituted at the 2 and 5 positions by aryl or heteroaryl groups. It would not have been obvious for the skilled man starting from this prior art to replace the aryl group at the 5-position by a piperidine group in order to make available compounds which act as positive allosteric modulators of mGluR5 receptors and which are useful for the treatment of CNS disorders.

### FIGURES

Figure 1 shows the effect of 10 µM of examples #1 and #4 of the present invention on primary cortical mGluR5-expressing cell cultures in the absence or in the presence of 300nM glutamate.
Figure 2 shows the representative compound # 1 of the invention significantly attenuated the increase in locomotor activity induced by amphetamine at doses of 30 mg/kg ip.

### DETAILED DESCRIPTION OF THE INVENTION

According to the present invention, there are provided new compounds of the general formula I

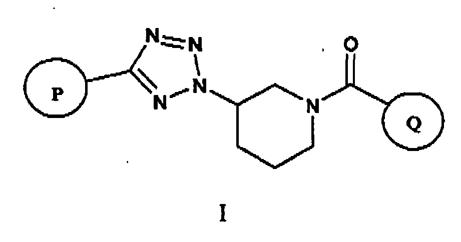

Or pharmaceutically acceptable salts, hydrates or solvates of such compounds Wherein P and Q are defined in claim 1.

The present invention includes both possible stereoisomers and includes not only racemic compounds but the individual enantiomers as well.

For the avoidance of doubt it is to be understood that in this specification "(C₁-C₆)" means a carbon group having 1, 2, 3, 4, 5 or 6 carbon atoms. "(C₀-C₆)" means a carbon group having 0, 1, 2, 3, 4, 5 or 6 carbon atoms.

In this specification "C" means a carbon atom.

In the above definition, the term "(C₁-C₆)alkyl" includes group such as methyl, ethyl, propyl, isopropyl, butyl, isobutyl, sec-butyl, tert-butyl, pentyl, isopentyl, neopentyl, tert-pentyl, hexyl or the like.

"Halogen" includes atoms such as fluorine, chlorine, bromine and iodine.

"Cycloalkyl" refers to an optionally substituted carbocycle containing no heteroatoms, includes mono-, bi-, and tricyclic saturated carbocycles, as well as fused ring systems. Such fused ring systems can include on ring that is partially or fully unsaturated such as a benzene ring to form fused ring systems such as benzo fused carbocycles. Cycloalkyl includes such fused ring systems as spirofused ring systems. Examples of cycloalkyl include cyclopropyl, cyclobutyl, cyclopentyl, cyclohexyl, decahydronaphthalene, adamantane, indanyl, fluorenyl, 1,2,3,4-tetrahydronaphthalene and the like.

"Solvate" refers to a complex of variable stoechiometry formed by a solute (e.g. a compound of formula I) and a solvent. The solvent is a pharmaceutically acceptable solvent as water preferably; such solvent may not interfere with the biological activity of the solute.

Specifically preferred compounds are:
(4-Fluoro-phenyl)-{(S)-3-[5-(4-fluoro-phenyl)-tetrazol-2-yl]-piperidin-1-yl}-methanone
(4-Fluoro-phenyl)-{3-[5-(4-fluoro-phenyl)-tetrazol-2-yl]-piperidin-1-yl}-methanone
(4-Fluoro-phenyl)-{3-[5-(2-fluoro-phenyl)-tetrazol-2-yl]-piperidin-l-yl}-methanone
(4-Fluoro-phenyl)-[(S)-3-(5-phenyl-tetrazol-2-yl)-piperidin-1-yl]-methanone
(4-Fluoro-phenyl)-[(R)-3-(5-phonyl-tetrazol-2-yl)-piperidin-1-yl]-methanone
{(S)-3-[5-(4-Chloro-phenyl)-tetrazol-2-yl]-piperidin-l-yl}-(4-fluoro-phenyl)-methanone
(4-Fluoro-phenyl)-{(S)-3-[5-(4-methoxy-phenyl)-tetrazol-2-yl]-piperidin-1-yl}-methanone
(4-Fluoro-phenyl)-{(S)-3-[5-(2-fluoro-phenyl)-tetrazol-2-yl]-piperidin-1-yl}-methanone
{(S)-3-[5-(2-Chloro-phenyl)-tetrazol-2-yl]-piperidm-1-yl}-(4-fluoro-phenyl)-methanone
(4-Fluoxo-phenyl)-{(R)-3-[5-(4-fluoro-phenyl)-tetrazol-2-yl]-piperidin-1-yl}-methanone
(4-Fluoro-phenyl)-[(S)-3-(5-pyridin-4-yl-tetrazol-2-yl)-piperidin-1-yl]-methanone
(4-Fluoro-phenyl)-[(S)-3-(5-pyridin-3-yl-tetrazol-2-yl)-piperidin-1-yl]-methanone
(4-Fluoro-phenyl)-[(S)-3-(5-pyridin-2-yl-tetrazol-2-yl)-piperidin-1-yl]-methanone
(4-Fluoro-phenyl)-{(S)-3-[5-(2-methoxy-phenyl)-tetrazol-2-yl]-piperidin-1-yl}-methanone
(4-Fluoro-phenyl)-[(S)-3-(5-p-tolyl-tetrazol-2-yl)-piperidin-1-yl]-methanone
(4-Fluoro-phenyl)-[(S)-3-(5-m-tolyl-tetrazol-2-yl)-piperidin-1-yl]-methanone
(4-Fluoro-phenyl)-{(S)-3-[5-(3-fluoro-phenyl)-tetrazol-2-yl]-piperidin-1-yl}-methanone
{(S)-3-[5-(4-Fluoro-phenyl)-tetrazal-2-yl]-piperidin-1-yl}-(5-methyl-isoxazol-4-yl)-methanone
(6-Fluoro-pyndm-3-yl)-[(S)-3-(5-phenyl-tetrazol-2-yl)-pipendm-1-yl]-methanone
(3,4-Difluoro-phenyl)-[(S)-3-(5-phenyl-tetrazol-2-yl)-piperidin-1-yl]-methanone
{(S)-3-[5-(4-Fluoro-phenyl)-tetrazol-2-yl]-piperidin-1-yl}-(6-fluoro-pyridin-3-yl)-methanone
(4-Fluoro-2-methyl-phenyl)-[(S)-3-(5-phenyl-tetrazol-2-yl)-piperidin-1-yl]-methanone
(4-Fluoro-1-methyl-phenyl)-{(S)-3-[5-(4-tluoro-phenyl)-tetrazol-2-yl]-piperidin-1-yl}-methanone
(3,4-Difluoro-phenyl)-((S)-3-[5-(4-fluoro-phenyl)-tetrazol-2-yl]-piperidin-1-yl)-methanone
(2,4-Difluoro-phenyl)-{(S)-3-[5-(4-fluoro-phenyl)-tetrazol-2-yl]-piperidin-1-yl}-methanone
(2,4-Difluoro-phenyl)-[(S)-3-(5-phenyl-tetrazol-2-yl)-piperidin-1-yl]-methanone
(5-Methyl-isoxazol-4-yl)-[(S)-3-(5-phenyl-tetrazol-2-yl)-piperidin-1-yl]-methanone

The compound (4-Fluoro-phenyl)-{3-[5-phenyl-tetrazol-2-yl]-piperidin-1-yl}-methanone was first disclosed in WO 2005/044797 (filed 4 November 2004) as "Example 68", and is disclaimed from the present invention.

The present invention relates to the pharmaceutically acceptable acid addition salts of compounds of the formula I or pharmaceutically acceptable carriers or excipients.

The present invention relates to compounds and compositions for treating or preventing a condition in a mammal, including a human, the treatment or prevention of which is affected or facilitated by the neuromodulatory effect of mGluR5 allosteric modulators and particularly positive allosteric modulators.

The present invention relates to compounds and compositions for treating or preventing peripheral and central nervous system disorders selected from the group consisting of: tolerance or dependence, anxiety, depression, psychiatric disease such as psychosis, inflammatory or neuropathic pain, memory impairment, Alzheimer's disease, ischemia, drug abuse and addiction.

The present invention relates to pharmaceutical compositions which provide from about 0.01 to 1000 mg of the active ingredient per unit dose. The compositions may be administered by any suitable route. For example orally in the form of capsules or tablets, parenterally in the form of solutions for injection, topically in the form of onguents or lotions, ocularly in the form of eye-lotion, rectally in the form of suppositories.

The pharmaceutical formulations of the invention may be prepared by conventional methods in the art; the nature of the pharmaceutical composition employed will depend on the desired route of administration. The total daily dose usually ranges from about 0.05 - 2000 mg.

### METHODS OF SYNTHESIS

Compounds of general formula I may be prepared by methods known in the art of organic synthesis as set forth in part by the following synthesis schemes. In all of the schemes described below, it is well understood that protecting groups for sensitive or reactive groups are employed where necessary in accordance with general principles of chemistry. Protecting groups are manipulated according to standard methods of organic synthesis (Green T.W. and Wuts P.G.M. (1991) Protecting Groups in Organic Synthesis, John Wiley et Sons). These groups are removed at a convenient stage of the compound synthesis using methods that are readily apparent to those skilled in the art. The selection of process as well as the reaction conditions and order of their execution shall be consistent with the preparation of compounds of formula I.
The compound of formula I may be represented as a mixture of enantiomers, which may be resolved into the individual pure R- or S-enantiomers. If for instance, a particular enantiomer of the compound of formula I is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group such as amino, or an acidic functional group such as carboxyl, this resolution may be conveniently performed by fractional crystallization from various solvents, of the salts of the compounds of formula I with optical active acid or by other methods known in the literature, e.g. chiral column chromatography. Resolution of the final product, an intermediate or a starting material may be performed by any suitable method known in the art as described by Eliel E.L., Wilen S.H. and Mander L.N. (1984) Stereochemistry of Organic Compounds, Wiley-Interscience.
Many of the heterocyclic compounds of formula I can be prepared using synthetic routes well known in the art (Katrizky A.R. and. Rees C.W. (1984) Comprehensive Heterocyclic Chemistry, Pergamon Press).
The product from the reaction can be isolated and purified employing standard techniques, such as extraction, chromatography, crystallization, distillation, and the like.

The compounds of formula **I** wherein W is a 3-substituted piperidine ring may be prepared according to the synthetic sequences illustrated in the Scheme 1.

Wherein
P and Q each independently is aryl or heteroaryl as described above
A represents -C(=O)-(C₀-C₂)alkyl-; -S(=O)₂-(C₀-C₂)alkyl-. 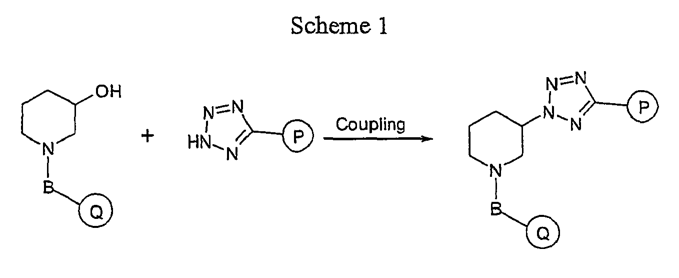
The precursor aryl-tetrazole derivatives are prepared according to synthetic routes well known in the art (Katrizky A.R. and Rees C.W. (1984) Comprehensive Heterocyclic Chemistry, Pergamon Press).
Aryl tetrazole can be alkylated with a 3-hydroxypiperidine derivative under Mitsunobu coupling conditions, as described in the literature (see for example: Synthetic Commun.; 26 ; 14 ; 1996 ; 2687-2694).
The reaction may be promoted by reagents known in the art of organic synthesis such as triphenylphosphine, tri-*n*-butylphosphine, substituted triarylphosphines or polymer-supported phosphines. Typically, reagents such as diethyl azodicarboxylate (DEAD), diisopropyl azodicarboxylate (DIAD), dimethyl azodicarboxylate or polymer-supported azodicarboxylate are present in the reaction mixture. The reaction is tipically carried out between 0° C and room temperature for a time in the range of about 2 hours up to 24 hours, in a suitable solvent (e.g. tetrahydrofuran, dioxane, dichloromethane, diethyl ether, dimethylformamide, toluene). Usually triphenylphosphine, the alcohol and the nucleophile are dissolved in the solvent and azodicarboxylate is added dropwise to the solution. Alternatively, azodicarboxylate and triphenylphosphine are reacted first to form the azodicarboxylate-triphenylphosphine adduct, followed by addition of the alcohol and nucleophile.

The compounds of formula I which are basic in nature can form a wide variety of different pharmaceutically acceptable salts with various inorganic and organic acids. These salts are readily prepared by treating the base compounds with a substantially equivalent amount of the chosen mineral or organic acid in a suitable organic solvent such as methanol, ethanol or isopropanol (see Stahl P.H., Wermuth C.G., Handbook of Pharmaceuticals Salts, Properties, Selection and Use, Wiley, 2002).

The following non-limiting examples 1 to 26 and 28 are intending to illustrate the invention. The physical data given for the compounds exemplified is consistent with the assigned structure of those compounds.

The compound of Example 27 is not an example claimed as the present invention.

### EXAMPLES

Unless otherwise noted, all starting materials were obtained from commercial suppliers and used without further purification.
Specifically, the following abbreviation may be used in the examples and throughout the specification.

| g (grams) | RT (retention time) |
|---|---|
| Mg (milligrams) | MeOH (methanol) |
| ml (millilitres) | |
| µl (microliters) | Hz (Hertz) |
| M (molar) | LCMS (Liquid Chromatography Mass Spectroscopy) |
| MHz (megahertz) | HPLC (High Pressure Liquid Chromatography) |
| mmol (millimoles) | NMR (Nuclear Magnetic Resonance) |
| min (minutes) | ¹H (proton) |
| AcOEt (ethyl acetate) | Na₂SO₄ (sodium sulphate) |
| K₂CO₃ (potassium carbonate) | MgSO₄ (magnesium sulphate) |
| PdCl₂(PPh₃)₂ (Bis(triphenylphosphine) palladium (II) dichloride | |
| CDCl₃ (deuterated chloroform) | HOBT (1-hydroxybenzotriazole) |
| EDCI.HCl (1-3(Dimethylaminopropyl)-3-ethylcarbodiimide, hydrochloride) | r.t. (Room Temperature) |
| EtOH (ethyl alcohol) | NaOH (sodium hydroxide) |
| % (percent) | h (hour) |
| DCM (dichloromethane) | HCl (hydrochloric acid) |
| DIEA (diisopropyl ethyl amine) | n-BuLi (n-butyllithium) |
| Mp (melting point) | THF (tetrahydrofuran) |

All references to brine refer to a saturated aqueous solution of NaCl. Unless otherwise indicated, all temperatures are expressed in °C (degrees Centigrade). All reactions are conducted not under an inert atmosphere at room temperature unless otherwise noted.
¹H NMR spectra were recorded on a Brucker 300MHz. Chemical shifts are expressed in parts per million (ppm, δ units). Coupling constants are in units of herts (Hz) Splitting patterns describe apparent multiplicities and are designated as s (singlet), d (doublet), t (triplet), q (quadruplet), quint (quintuplet), m (multiplet).
The microwave oven used is an apparatus from Biotage (Optimizer^{™}) equipped with an internal probe that monitors reaction temperature and pressure, and maintains the desired temperature by computer control.

LCMS were recorded under the following conditions:
Method A): Waters Alliance 2795 HT Micromass ZQ. Column Waters XTerra MS C18 (50x4.6 mm, 2.5µm). Flow rate 1 ml/min Mobile phase: A phase = water/CH₃CN 95/5 + 0.05% TFA, B phase = water/CH₃CN = 5/95 + 0.05% TFA. 0-1 min (A: 95%, B: 5%), 1-4 min (A: 0%, B: 100%), 4-6 min (A: 0%, B: 100%), 6-6.1 min (A: 95%, B: 5%). T= 35°C; UV detection: Waters Photodiode array 996, 200-400nm.
Method B): Waters Alliance 2795 HT Micromass ZQ. Column Waters XTerra MS C18 (50x4.6 mm, 2.5µm). Flow rate 1.2 ml/min Mobile phase: A phase = water/CH₃CN 95/5 + 0.05% TFA, B phase = water/CH₃CN = 5/95 + 0.05% TFA. 0-0.8 min (A: 95%, B: 5%), 0.8-3.3 min (A: 0%, B: 100%), 3.3-5 min (A: 0%, B: 100%), 5-5.1 min (A: 95%, B: 5%). T= 35°C; UV detection: Waters Photodiode array 996, 200-400nm.
Method C): Waters Alliance 2795 HT Micromass ZQ. Column Waters Symmetry C18 (75x4.6 mm, 3.5µm). Flow rate 1 ml/min Mobile phase: A phase = water/CH₃CN 95/5 + 0.05% TFA, B phase = water/CH₃CN = 5/95 + 0.05% TFA.
   0-0.1 min (A: 95%, B: 5%), 1-11 min (A: 0%, B: 100%), 11-12 min (A: 0%, B: 100%), 12-12.1 min (A: 95%, B: 5%). T= 35°C; UV detection: Waters Photodiode array 996, 200-400nm.
Method D): Waters Alliance 2795 HT Micromass ZQ. Column Waters Symmetry C18 (75x4.6 mm, 3.5µm). Flow rate 1.5 ml/min Mobile phase: A phase = water/CH₃CN 95/5 + 0.05% TFA, B phase = water/CH₃CN = 5/95 + 0.05% TFA.
   0-0.5 min (A: 95%, B: 5%), 0.5-7 min (A: 0%, B: 100%), 7-8 min (A: 0%, B: 100%), 8-8.1 min (A: 95%, B: 5%). T= 35°C; UV detection: Waters Photodiode array 996, 200-400nm.
Method E): Pump 515, 2777 Sample Manager, Micromass ZQ Single quadrupole (Waters). Column 2.1x50mm stainless steel packed with 3.5µm SunFire RP C-18 (Waters); flow rate 0.25 ml/min splitting ratio MS :waste/ 1:4; mobile phase: A phase = water/acetonitrile 95/5 + 0.1 % TFA, B phase = water/acetonitrile 5/95 + 0.1% TFA.
   0-1.0min (A: 98%, B: 2%), 1.0-5.0mien (A: 0%, B: 100%), 5.0-9.0min (A: 0%, B: 100%), 9.1.0-12main (A: 98%, B: 2%); UV detection wavelength 254 nm; Injection volume: 5 µl
Method F): Waters Alliance 2795 HT Micromass ZQ. Column Waters XTerra MS C18 (50x4.6 mm, 2.5µm). Flow rate 1.2 ml/min. Mobile phase: A phase = water/CH₃CN 95/5 + 0.05% TFA, B phase = water/CH₃CN = 5/95 + 0.05% TFA. 0-1.7 min (A: 95%, B: 5%), 1.7-4.2 min (A: 0%, B: 100%), 4.2-5.0 min (A: 0%, B: 100%), 5-5.1 min (A: 95%, B: 5%). T= 35°C; UV detection: Waters Photodiode array 996, 200-400nm.

All mass spectra were taken under electrospray ionisation (ESI) methods.

Most of the reactions were monitored by thin-layer chromatography on 0.25mm Merk silica gel plates (60F-254), visualized with UV light. Flash column chromatography was performed on silica gel (220-440 mesh, Fluka) or prepacked silica gel cartridge columns using a Biotage flash unit.
Melting point determination was performed on a Buchi B-540 apparatus.

### Example 1

### (4-Fluoro-phenyl)-{(S)-3-[5-(4-fluoro-phenyl)-tetrazol-2-yl]-piperidin-1-yl}-methanone

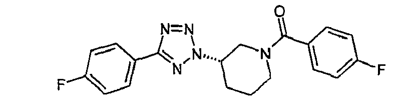

### 1 (A) (4-Fluoro-phenyl)-((R)-3-hydroxy-piperidin-1-yl)-methanone

A mixture of (R)-3-hydroxy piperidine hydrochloride (0.2 g, 1.45 mmol), 4-fluoro benzoic acid (0.204 g, 1.45 mmol), EDC.HCl (0.42 g, 2.18 mmol), HOBT (0.196 g, 1.45 mmol), triethylamine (320 uL, 4.36 mmol) in dichloromethane (10 mL) was stirred under nitrogen atmosphere overnight at room temperature. The reaction mixture was diluted with dichloromethane (20 mL) and washed subsequently with 0.1N HCl (2 times), 0.1N NaOH (2 times) and then with brine. The organic layer was dried over sodium sulphate and evaporated under reduced pressure to give a pale yellow oil (275 mg), which was used for the next step without further purification. Yield: 85%; [α]_{D}²⁰ =-8.7° (c=0.615, CHCl₃); LCMS (RT): 3.1 min (Method D); MS (ES+) gave m/z: 224.0.
¹H-NMR (CDCl₃); δ (ppm): 7.43 (dd, 2H); 7.08 (dd, 2H); 4.00-3.14 (m br, 5H); 2.27 (s br, 1H); 1.98-1.76 (m, 2H); 1.74-1.55 (m, 2H).

### 1(B) (4-Fluoro-phenyl)-{(S)-3-[5-(4-fluoro-phenyl)-tetrazol-2-yl]-piperidin-1-yl}-methanone

Diisopropylazadicarboxylate (DIAD, 137 uL, 0.7 mmol) was added dropwise at room temperature to a mixture of 4-fluorophenyl tetrazole (74 mg, 0.45 mmol), (4-fluoro-phenyl)-((R)-3-hydroxy-piperidin-1-yl)-methanone (100 mg, 0.45 mmol) and triphenylphosphine (184 mg, 0.7 mmol) in dichloromethane (10 mL). After stirring overnight at ambient temperature under nitrogen atmosphere, the solvent was evaporated under reduced pressure and the residue was purified by flash column chromatography (silica gel, eluent gradient: from ethyl acetate/hexane 3:7 to ethyl acetate/hexane 7:3). A second flash column chromatography (silica gel, eluent: DCM/MeOH 98:2) was performed on the residue; the crude material thus recovered was then dissolved in a mixture of hexane and DCM (99:1) and passed through a silica gel cartridge (Isolute Flash II 2 g, eluent gradient: starting with hexane/diethyl ether 8:2, then with hexane/diethyl ether 6:4, then with DCM/MeOH 96:4).
The title compound was obtained pure as a pale solid (45 mg).
Yield: 27%; (pale solid); mp=109-110°C; [α]_{D}²⁰ = +91.9° (c=1.1, CHCl₃); LCMS (RT): 7.43 min (Method: E); MS (ES+) gave m/z: 370.1.
¹H-NMR (DMSO-d₆), δ (ppm): 8.08 (dd, 2H); 7.43 (dd, 2H); 7.35 (dd, 2H); 7.19 (dd, 2H); 5.08 (m, 1H); 4.30 (dd, 1H); 3.86 (dd, 1H); 3.69 (ddd, 1H); 3.47 (ddd, 1H); 2.47-2.28 (m, 2H); 1.96 (m, 1H); 1.81-1.66 (m, 1H).

### Example 2

### (4-Fluoro-phenyl)-{3-[5-(4-fluoro-phenyl)-tetrazol-2-yl]-piperidin-1-yl}-methanone

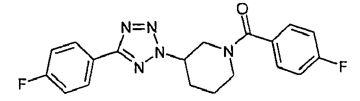

### 2(A) (4-Fluoro-phenyl)-(3-hydroxy-piperidin-1-yl)-methanone

The title compound was prepared starting from (±)-3-hydroxy piperidine hydrochloride, according to the same experimental procedure described in Example 1 (A).
Yield: 82%; LCMS (RT): 2.5 min (Method B); MS (ES+) gave m/z: 224.1.

### 2(B) (4-Fluoro-phenyl)-{3-[5-(4-fluoro-phenyl)-tetrazol-2-yl]-piperidin-1-y}-methanone

A mixture of diisopropylazadicarboxylate (DIAD, 137 uL, 0.7 mmol), 4-fluorophenyl tetrazole (74 mg, 0.45 mmol), (4-fluoro-phenyl)-(3-hydroxy-piperidin-1-yl)-methanone (100 mg, 0.45 mmol) and solid supported triphenylphosphine (PS-PPh₃, ex Argonaut Technologies, loading 1.6 mmol/g, 625 mg, 1.0 mmol) in dichloromethane (5 mL) was heated under microwave irradiation for 30 min at 70°C.
The resin was filtered off and washed with dichloromethane. The filtrate was washed with 1M HCl and then with 1M NaOH and the organic layer was dried over magnesium sulphate and evaporated under reduced pressure. The residue was purified by flash column chromatography (silica gel, eluent gradient: from DCM to DCM/MeOH 99:1). The crude material thus recovered was then dissolved in a mixture of hexane and DCM (99:1) and passed through a silica gel cartridge (Isolute Flash II 2 g, eluent gradient: starting with hexane, then with hexane/diethyl ether 6:4, then with DCM/MeOH 98:2).
The title compound was obtained pure as a white solid (25 mg).
Yield: 15%; (white solid); mp=137-139°C; LCMS (RT): 7.45 min (Method: E); MS (ES+) gave m/z: 370.1.
¹H-NMR (CDCl₃), δ (ppm): 8.16 (m, 2H); 7.41 (m, 2H); 7.17 (dd, 2H); 7.09 (dd, 2H); 4.90 (m, 1H); 4.79-3.83 (m br, 2H); 3.77 (dd, 1H); 3.35 (dd, 1H); 2.55-2.30 (m, 2H); 2.04 (m, 1H); 1.74 (m, 1H).

### Example 3

### (4-Fluoro-phenyl)-{3-[5-(2-fluoro-phenyl)-tetrazol-2-yl]-piperidin-1-yl}-methanone

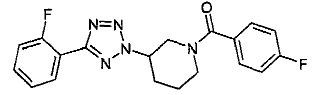

The title compound was prepared following the experimental procedure described in Example 2(B), starting from (4-fluoro-phenyl)-(3-hydroxy-piperidin-1-yl)-methanone and 2-fluorophenyl tetrazole.
Yield: 15% (colourless gum); LCMS (RT): 7.11 min (Method: E); MS (ES+) gave m/z: 369.9.
¹H-NMR (DMSO-d₆), δ (ppm): 8.01 (ddd, 1H); 7.59 (m, 1H); 7.47-7.33 (m, 4H); 7.19 (dd, 2H); 5.12 (m, 1H); 4.31 (dd, 1H); 3.86 (dd, 1H); 3.69 (m, 1H); 3.47 (ddd, 1H); 2.48-2.27 (m, 2H); 1.97 (m, 1H); 1.81-1.67 (m, 1H).

### Example 4

### (4-Fluoro-phenyl)-[(S)-3-(5-phenyl-tetrazol-2-yl)-piperidin-1-yl]-methanone

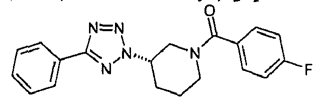

A mixture of diisopropylazadicarboxylate (DIAD, 177 uL, 0.9 mmol), phenyl tetrazole (66 mg, 0.45 mmol), (4-fluorophenyl)-((R)-3-hydroxy-piperidin-1-yl)-methanone (100 mg, 0.45 mmol) and solid supported triphenylphosphine (PS-PPh₃, ex Argonaut Technologies, loading 1.6 mmol/g, 625 mg, 1.0 mmol) in toluene (5 mL) was heated under microwave irradiation for 20 min at 100°C.
The resin was filtered off, washed with dichloromethane and the filtrate was evaporated under reduced pressure. The residue was first purified by preparative HPLC. The crude material thus recovered was then dissolved in toluene and passed through a silica gel cartridge (Isolute Flash II 2 g, eluent gradient: starting with toluene, then with hexane/diethyl ether 7:3, then with DCM/MeOH 98:2).
The title compound was obtained pure as a colourless gum (32 mg).
Yield: 20%; (colourless gum); LCMS (RT): 7.27 min (Method: E); MS (ES+) gave m/z: 352.1.
¹H-NMR (DMSO-d₆), δ (ppm): 8.04 (m, 2H); 7.61-7.52 (m, 3H); 7.44 (dd, 2H); 7.22 (dd, 2H); 5.11 (m, 1H); 4.29 (m, 1H); 3.84 (dd, 1H); 3.67 (m, 1H); 3.46 (m, 1H); 2.47-2.26 (m, 2H); 1.93 (m, 1H); 1.73 (m, 1H).

### Example 5

### (4-Fluoro-phenyl)-[(R)-3-(S-phenyl-tetrazol-2-yl)-piperidin-1-yl]-methanone

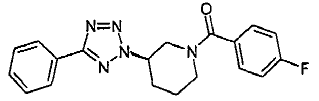

### 5(A) (4-Fluoro-phenyl)-((S)-3-hydroxy-piperidin-1-yl)-methanone

The title compound was prepared starting from (S)-3-hydroxy piperidine hydrochloride, according to the same experimental procedure described in Example 1 (A).
Yield: 94%; LCMS (RT): 2.5 min (Method D); MS (ES+) gave m/z: 224.1.

### 5(B) (4-Fluoro-phenyl)-[(R)-3-(5-phenyl-tetrazol-2-yl)-piperidin-1-yl]-methanone

A mixture of diisopropylazadicarboxylate (DIAD, 141 uL, 0.72 mmol), phenyl tetrazole (53 mg, 0.36 mmol), (4-fluorophenyl)-((S)-3-hydroxy-piperidin-1-yl)-methanone (80 mg, 0.36 mmol) and solid supported triphenylphosphine (PS-PPh₃, ex Argonaut Technologies, loading 1.6 mmol/g, 625 mg, 1.0 mmol) in toluene (5 mL) was heated under microwaves irradiation for 20 min at 100°C.
The resin was filtered off, washed with dichloromethane and the filtrate was evaporated under reduced pressure. The residue was first purified by preparative HPLC. The crude material thus recovered was then dissolved in toluene and passed through a silica gel cartridge (Isolute Flash II 2 g, eluent gradient: starting with toluene, then with hexane/diethyl ether 7:3, then with DCM/MeOH 98:2).
The title compound was obtained pure as a colourless gum (31 mg).
Yield: 25%; (colourless gum); LCMS (RT): 7.27 min (Method: E); MS (ES+) gave m/z: 352.1.
¹H-NMR (DMSO-d₆), δ (ppm): 8.04 (m, 2H); 7.61-7.52 (m, 3H); 7.44 (dd, 2H); 7.22 (dd, 2H); 5.11 (m, 1H); 4.29 (m, 1H); 3.84 (dd, 1H); 3.67 (m, 1H); 3.46 (m, 1H); 2.47-2.26 (m, 2H); 1.93 (m, 1H); 1.73 (m, 1H).

### Example 6

### {(S)-3-[5-(4-Chloro-phenyl)-tetrazol-2-yl]-piperidin-1-yl}-(4-fluoro-phenyl)-methanone

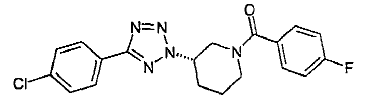

The title compound was prepared following the same experimental procedure described in Example 5(B), starting from 4-chlorophenyl tetrazole and (4-fluorophenyl)-((R)-3-hydroxy-piperidin-1-yl)-methanone (prepared as described in Example 1 (A)).
Yield: 9%; (colourless gum); LCMS (RT): 7.8 min (Method: E); MS (ES+) gave m/z: 386.
¹H-NMR (DMSO-d₆), δ (ppm): 8.05 (d, 2H); 7.63 (d, 2H); 7.43 (dd, 2H); 7.23 (dd, 2H); 5.12 (m, 1H); 4.29 (m, 1H); 3.84 (m, 1H); 3.66 (m, 1H); 3.46 (m, 1H); 2.47-2.25 (m, 2H); 1.92 (m, 1H); 1.72 (m, 1H).

### Example 7

### (4-Fluoro-phenyl)-{(S)-3-[5-(4-methoxy-phenyl)-tetrazol-2-yl]-piperidin-1-yl}-methanone

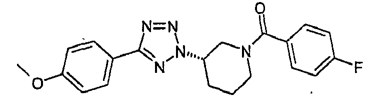

The title compound was prepared following the same experimental procedure described in Example 5(B), starting from 4-methoxyphenyl tetrazole and (4-fluorophenyl)-((R)-3-hydroxy-piperidin-1-yl)-methanone (prepared as described in Example 1 (A)).
Yield: 8%; (colourless gum); LCMS (RT): 7.23 min (Method: E); MS (ES+) gave m/z: 382.1.
¹H-NMR (DMSO-d₆), δ (ppm): 7.97 (d, 2H); 7.43 (dd, 2H); 7.23 (dd, 2H); 7.11 (d, 2H); 5.07 (m, 1H); 4.28 (m, 1H); 3.85 (s, 3H); 3.81 (dd, 1H); 3.66 (m, 1H); 3.45 (m, 1H); 2.47-2.24 (m, 2H); 1.92 (m, 1H); 1.72 (m, 1H).

### Example 8

### (4-Fluoro-phenyl)-{(S)-3-[5-(2-fluoro-phenyl)-tetrazol-2-yl]-pipendm-1-yl}-methanone

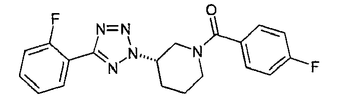

The title compound was prepared following the same experimental procedure described in Example 5(B), starting from (4-fluoro-phenyl)-((R)-3-hydroxy-piperidin-1-yl)-methanone and 2-fluorophenyl tetrazole.
Yield: 19%; (colourless gum); LCMS (RT): 7.14 min (Method: E); MS (ES+) gave m/z: 370.0.
¹H-NMR (DMSO-d₆), δ (ppm): 8.03 (ddd, 1H); 7.61 (m, 1H); 7.47-7.36 (m, 4H); 7.22 (dd, 2H); 5.15 (m, 1H); 4.30 (m, 1H); 3.84 (dd, 1H); 3.67 (m, 1H); 3.45 (m, 1H); 2.48-2.25 (m, 2H); 1.93 (m, 1H); 1.73 (m, 1H).

### Example 9

### {(S)-3-[5-(2-Chloro-phenyl)-tetrazol-2-yl]-piperidin-1-yl}-(4-fluoro-phenyl)-methanone

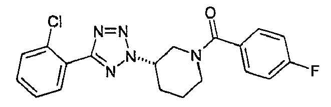

The title compound was prepared following the same experimental procedure described in Example 5(B), starting from 2-chlorophenyl tetrazole and (4-fluorophenyl)-((R)-3-hydroxy-piperidin-1-yl)-methanone (prepared as described in Example 1 (A)).
Yield: 22%; (colourless gum); LCMS (RT): 7.37 min (Method: E); MS (ES+) gave m/z: 386.0.
¹H-NMR (DMSO-d₆), δ (ppm): 7.88 (dd, 1H); 7.66 (dd, 1H); 7.58 (ddd, 1H); 7.52 (ddd, 1H); 7.43 (dd, 2H); 7.22 (dd, 1H); 5.16 (m, 1H); 4.30 (m, 1H); 3.84 (dd, 1H); 3.65 (m, 1H); 3.46 (m, 1H); 2.48-2.25 (m, 2H); 1.93 (m, 1H); 1.73 (m, 1H).

### Example 10

### (4-Fluoro-phenyl)-{(R)-3-[5-(4-fluoro-phenyl)-tetrazol-2-yl]-piperidin-1-yl}-methanone

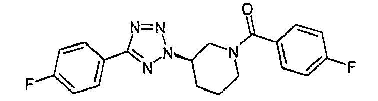

A mixture of diisopropylazadicarboxylate (DIAD, 177 uL, 0.9 mmol), 4-fluorophenyl tetrazole (74 mg, 0.45 mmol), (4-fluoro-phenyl)-((S)-3-hydroxy-piperidin-1-yl)-methanone (100 mg, 0.45 mmol) and solid supported triphenylphosphine (PS-PPh₃, ex Argonaut Technologies, loading 1.6 mmol/g, 625 mg, 1.0 mmol) in toluene (5 mL) was heated under microwave irradiation for 30 min at 90°C.
The resin was filtered off, washed with dichloromethane and the filtrate was evaporated under reduced pressure. The residue was first purified by preparative HPLC. The crude material thus recovered was then dissolved in toluene and passed through a silica gel cartridge (Isolute Flash II 2 g, eluent gradient: starting with toluene, then with hexane/diethyl ether 7:3, then with DCM/MeOH 98:2).
The title compound was obtained pure as a colourless gum (31 mg).
Yield: 10%; (colourless gum); LCMS (RT): 7.33 min (Method: E); MS (ES+) gave m/z: 370.0.
¹H-NMR (CDCl₃), δ (ppm): 8.12 (dd, 2H); 7.41 (dd, 2H); 7.17 (dd, 2H); 7.08 (dd, 2H); 4.89 (m, 1H); 4.50 (m, 1H); 3.96 (m, 1H); 3.78 (dd, 1H); 3.37 (ddd, 1H); 2.54-2.29 (m, 2H); 2.06 (m, 1H); 1.75 (m, 1H).

### Example 11

### (4-Fluoro-phenyl)-[(S)-3-(5-pyridin-4-yl-tetrazol-2-yl)-piperidin-1-yl]-methanone

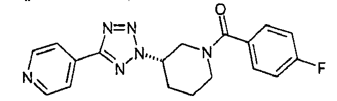

A mixture of diisopropylazadicarboxylate (DIAD, 141 uL, 0.72 mmol), 4-pyridyl tetrazole (53 mg, 0.36 mmol), (4-fluorophenyl)-((R)-3-hydroxy-piperidin-1-yl)-methanone (80 mg, 0.36 mmol) and solid supported triphenylphosphine (PS-PPh₃, ex Argonaut Technologies, loading 1.6 mmol/g, 625 mg, 1.0 mmol) in DCM (5 mL) was stirred at room temperature overnight and then heated under microwave irradiation for 15 min at 80°C.
The resin was filtered off, washed with dichloromethane and the filtrate was evaporated under reduced pressure. The residue was dissolved in DCM and loaded onto a SCX cartridge (eluent gradient: starting from DCM, then with MeOH, then eluting with 10% NH₃ in MeOH). The crude product thus recovered was passed through a silica gel cartridge (Isolute Flash Si 2g, eluent gradient: starting from DCM, then with DCM/MeOH 99.5:0.5, then eluting with DCM/MeOH 98.5:1.5).
The title compound was obtained pure as a colourless gum (38 mg).
Yield: 30%; (colourless gum); LCMS (RT): 5.68 min (Method: E); MS (ES+) gave m/z: 353.1.
¹H-NMR (CDCl₃), δ (ppm): 8.78 (d, 2H); 8.06 (m, 2H); 7.42 (dd, 2H); 7.09 (dd, 2H); 4.96 (m, 1H); 4.54 (m, 1H); 3.96 (m, 1H); 3.81 (dd, 1H); 3.38 (ddd, 1H); 2.57-2.35 (m, 2H); 2.06 (m, 1H); 1.76 (m, 1H).

### Example 12

### (4-Fluoro-phenyl)-[(S)-3-(5-pyridin-3-yl-tetrazol-2-yl)-piperidin-1-yl]-methanone

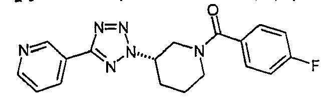

The title compound was prepared following the same experimental procedure described in Example 11, starting from 3-pyridinyl tetrazole and (4-fluoro-phenyl)-((R)-3-hydroxy-piperidin-1-yl)-methanone (prepared as described in Example 1 (A)).
Yield: 27%; (colourless gum); LCMS (RT): 5.75 min (Method: E); MS (ES+) gave m/z: 353.1.
¹H-NMR (CDCl₃), δ (ppm): 9.36(d br, 1H); 8.72(dd, 1H); 8.43(ddd, 1H); 7.47-7.38(m, 3H); 7.09(dd, 2H); 4.94(m, 1H); 4.53(m, 1H); 3.97(m, 1H); 3.80(dd, 1H) 3.37(ddd, 1H); 2.57-2.32(m, 2H); 2.06(m, 1H); 1.75(m; 1H).

### Example 13

### (4-Fluoro-phenyl)-[(S)-3-(5-pyidin-2-yl-tetrazol-2-yl)-piperidin-1-yl]-methanone

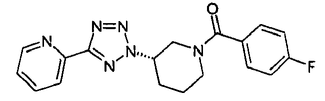

The title compound was prepared following the same experimental procedure described in Example 11, starting from (4-fluoro-phenyl)-((R)-3-hydroxy-piperidin-1-yl)-methanone and 2-pyridinyl tetrazole.
Yield: 31%; (colourless gum); LCMS (RT): 6.22 min (Method: E); MS (ES+) gave m/z: 353.1.
¹H-NMR (CDCl₃), δ (ppm): 8.78 (m, 1H); 8.22 (ddd, 1H); 7.84 (ddd, 1H); 7.47-7.34 (m, 3H); 7.07 (dd, 2H); 4.96 (m, 1H); 4.51 (m, 1H); 4.02 (m, 1H); 3.82 (dd, 1H); 3.30 (ddd, 1H); 2.55-2.34 (m, 2H); 2.05 (m, 1H); 1.75 (m, 1H).

### Example 14

### (4-Fluoro-phenyl)-{(S)-3-[5-(2-methoxy-phenyl)-tetrazol-2-yl]-piperidin-1-yl}-methanone

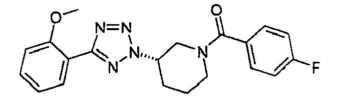

The title compound was prepared following the same experimental procedure described in Example 5(B), starting from (4-fluoro-phenyl)-((R)-3-hydroxy-piperidin-1-yl)-methanone and 2-methoxyphenyl tetrazole.
Yield: 12%; (colourless gum); LCMS (RT): 7.1 min (Method: E); MS (ES+) gave m/z: 382.1.
¹H-NMR (CDCl₃), δ (ppm): 7.91 (d, 1H); 7.49-7.36 (m, 3H); 7.13-7.02 (m, 4H); 4.94 (m, 1H); 4.60 (m, 1H); 4.01 (m, 1H); 3.92 (s, 3H); 3.81 (dd, 1H); 3.36 (ddd, 1H); 2.54-2.31 (m, 2H); 2.07 (m, 1H); 7.17 (m, 1H).

### Example 15

### (4-Fluoro-phenyl)-[(S)-3-(5-p-tolyl-tetrazol-2-yl)-piperidin-1-yl]-methanone

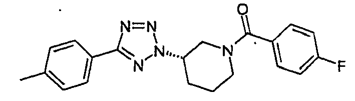

The title compound was prepared following the same experimental procedure described in Example 5(B), starting from (4-fluorophenyl)-((R)-3-hydroxy-piperidin-1-yl)-methanone and 4-tolyl tetrazole.
Yield: 6%; (colourless gum); LCMS (RT): 7.50 min (Method: E); MS (ES+) gave m/z: 366.2.
¹H-NMR (CDCl₃), δ (ppm): 8.00 (d, 2H); 7.41 (dd, 2H); 7.29 (d, 2H); 7.09 (dd, 2H); 4.88 (m, 1H); 4.51 (m, 1H); 3.99 (m, 1H); 3.76 (m, 1H); 3.36 (m, 1H); 2.43 (m, 2H); 2.42 (s, 3H); 2.04 (m, 1H); 1.75 (m, 1H).

### Example 16

### (4-Fluoro-phenyl)-[(S)-3-(5-m-tolyl-tetrazol-2-yl)-piperidin-1-yl]-methanone

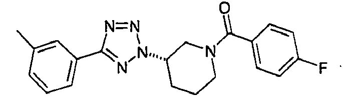

The title compound was prepared following the same experimental procedure described in Example 5(B), starting from (4-fluorophenyl)-((R)-3-hydroxy-piperidin-1-yl)-methanone and 3-tolyl tetrazole.
Yield: 8%; (colourless gum); LCMS (RT): 7.60 min (Method: E); MS (ES+) gave m/z: 366.2.
¹H-NMR (CDCl₃), δ (ppm): 7.95 (s, 1H); 7.92 (d, 1H); 7.41 (dd, 2H); 7.36 (dd, 1H); 7.27 (dd, 1H); 7.08 (dd, 2H); 4.89 (m, 1H); 4.49 (m, 1H); 3.97 (m, 1H); 3.80 (dd, 1H); 3.37 (ddd, 1H); 2.54-2.32 (m, 2H); 2.44 (s, 3H); 2.06 (m, 1H); 1.75 (m, 1H).

### Example 17

### (4-Fluoro-phenyl)-{(S)-3-[5-(3-fluoro-phenyl)-tetrazol-2-yl]-piperidin-1-yl}-methanone

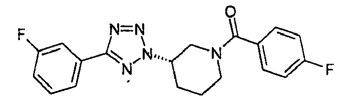

The title compound was prepared following the same experimental procedure described in Example 5(B), starting from (4-fluoro-phenyl)-((R)-3-hydroxy-piperidin-1-yl)-methanone and 3-fluorophenyl-tetrazole.
Yield: 37% (colourless oil); LCMS (RT): 7.63 min (Method: E); MS (ES+) gave m/z: 370.1.
¹H-NMR (DMSO-d₆), δ (ppm): 7.89 (ddd, 1H); 7.76 (ddd, 1H); 7.62 (ddd, 1H); 7.43 (dd, 2H); 7.36 (m, 1H); 7.21 (dd, 2H); 5.11 (m, 1H); 4.29 (dd, 1H); 3.87 (dd, 1H); 3.67 (m, 1H); 3.47 (m, 1H); 2.48-2.27 (m, 2H); 1.93 (m, 1H); 1.73 (m, 1H).

### Example 18

### {(S)-3-[5-(4-Fluoro-phenyl)-tetrazol-2-yl]-piperidin-1-yl}-(5-methyl-isoxazol-4-yl)-methanone

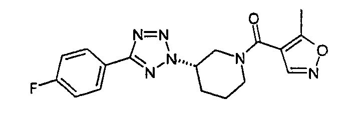

### 18(A) ((R)-3-Hydroxy-piperidin-1-yl)-(5-methyl-isoxazol-4-yl)-methanone

The title compound was prepared starting from (R)-3-hydroxy-piperidine hydrochloride and 5-methyl-isoxazole-4-carboxylic acid, according to the same experimental procedure described in Example 1(A).
Yield: 46%; LCMS (RT): 1.21 min (Method: F); MS (ES+) gave m/z: 211.1.

### 18(B) {(S)-3-[5-(4-Fluoro-phenyl)-tetrazol-2-yl]-piperidin-1-yl}-(5-methyl-isoxazol-4-yl)-methanone

The title compound was prepared following the same experimental procedure described in Example 5(B), starting from ((R)-3-Hydroxy-piperidin-1-yl)-(5-methyl-isoxazol-4-yl)-methanone and 4-fluorophenyl tetrazole.
Yield: 13% (colourless oil); LCMS (RT): 6.32 min (Method: E); MS (ES+) gave m/z: 357.2.
¹H-NMR (DMSO-d₆, 373K), δ (ppm): 8.50(s, 1H); 8.08(dd, 2H); 7.35(dd, 2H); 5.08(m, 1H); 4.32(dd, 1H); 3.91(dd, 1H); 3.73(ddd, 1H); 3.53(ddd, 1H); 2.44(s, 3H); 2.44-2.29(m, 2H); 2.03-1.90(m, 1H); 1.83-1.67(m, 1H).

### Example 19

### (6-Fluoro-pyridin-3-yl)-[(S)-3-(5-phenyl-tetrazol-2-yl)-piperidin-1-yl]-methanone

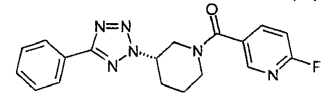

### 19(A) (6-Fluoro-pyridin-3-yl)-((R)-3-hydroxy-piperidin-1-yl)-methanone

The title compound was prepared starting from (R)-3-hydroxy-piperidine hydrochloride and 2-fluoropyridine-5-carboxylic acid, according to the same experimental procedure described in Example 1 (A).
Yield: 85%; LCMS (RT): 1.42 min (Method: B); MS (ES+) gave m/z: 225.0.

### 19(B) (6-Fluoro-pyridin-3-yl)-[(S)-3-(5-phenyl-tetrazol-2-yl)-piperidin-1-yl]-methanone

The title compound was prepared following the same experimental procedure described in Example 5(B), starting from (6-fluoro-pyridin-3-yl)-((R)-3-hydroxy-piperidin-1-yl)-methanone and phenyl-tetrazole.
The title compound was purified by flash chromatography (eluent gradient: starting DCM, then with DCM/MeOH 98:2) and then by silica gel cartridge (Isolute Flash II 5 g, eluent gradient: starting with toluene, then hexane/diethyl ether 7:3, then with DCM/MeOH 98:2).
Yield: 34%; (colourless gum); LCMS (RT): 6.30 min (Method: C); MS (ES+) gave m/z: 353.2.
¹H-NMR (DMSO-d₆), δ (ppm): 8.28 (s br, 1H); 8.08-7.93 (m, 3H); 7.61-7.50 (m, 3H); 7.17 (dd, 1H); 5.12 (m, 1H); 4.31 (m, 1H); 3.91 (dd, 1H); 3.70 (m, 1H); 3.52 (m, 1H); 2.49-2.27 (m, 2H); 1.93 (m, 1H); 1.77 (m, 1H).

### Example 20

### (3,4-Difluoro-phenyl)-[(S)-3-(5-phenyl-tetrazol-2-yl)-piperidin-1-yl]-methanone

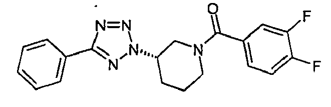

### 20(A) (3,4-Difluoro-phenyl)-((R)-3-hydroxy-piperidin-1-yl)-methanone

To a solution of (R)-3-hydroxy-piperidine hydrochloride (0.2 g, 1.45 mmol) and triethylamine (415 uL, 3.0 mmol) in dichloromethane (10 mL), under nitrogen atmosphere, 3,4-difluorobenzoyl chloride (183 uL, 1.45 mmol) was added slowly at 0°C. The reaction mixture was allowed to warm at room temperature and stirred for 3h. The solution was treated with IN HCl (10 mL), the phases were separated and the organics were washed with IN NaOH and with brine. The organic layer was dried over sodium sulphate and evaporated under reduced pressure to afford (3,4-difluorophenyl)-((R)-3-hydroxy-piperidin-1-yl)-methanone.
Yield: 80%; LCMS (RT): 2.6 min (Method: B); MS (ES+) gave m/z: 242.0.

### 20(B) (3,4-Difluoro-phenyl)-[(S)-3-(5-phenyl-tetrazol-2-yl)-piperidin-1-yl]-methanone

The title compound was prepared following the same experimental procedure described in Example 5(B), starting from (3,4-difluoro-phenyl)-((R)-3-hydroxy-piperidin-1-yl)-methanone and phenyl-tetrazole.
Yield: 39% (colourless gum); LCMS (RT): 6.88 min (Method: C); MS (ES+) gave m/z: 370.2.
¹H-NMR (DMSO-d₆), δ (ppm): 8.05 (m, 2H); 7.61-7.51 (m, 3H); 7.49-7.37 (m, 2H); 7.24 (m, 1H); 5.12 (m, 1H); 4.28 (m, 1H); 3.86 (dd, 1H); 3.67 (m, 1H); 3.48 (m, 1H); 2.48-2.26 (m, 2H); 1.93 (m, 1H); 1.75 (m, 1H).

### Example 21

### {(S)-3-[5-(4-Fluoro-phenyl)-tetrazol-2-yl]-piperidm-1-yl}-(6-fluoro-pyridin-3-yl)-methanone

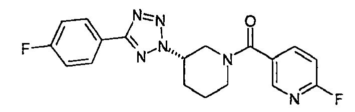

Diisopropylazadicarboxylate (DIAD, 174 uL, 0.9 mmol) was added dropwise at 0°C to a mixture of 4-fluorophenyl tetrazole (74 mg, 0.45 mmol), (6-ffuoro-pyridin-3-yl)-((R)-3-hydroxy-piperidin-1-yl)-methanone (100 mg, 0.45 mmol, prepared as described in 19 (A)) and solid supported triphenylphosphine (PS-PPh₃, ex Argonaut Technologies, loading 2.4 mmol/g, 560 mg, 1.35 mmol) in dichloromethane (4 mL). After stirring overnight at room temperature under nitrogen atmosphere, the resin was filtered off, washed with dichloromethane and the solvent was evaporated under reduced pressure. The crude material thus recovered was then dissolved in toluene and passed through a silica gel cartridge (Isolute Flash II 5 g, eluent gradient: starting with toluene, then hexane/diethyl ether 7:3, then with DCM, then with DCM/MeOH 98:2). The crude was then purified by preparative HPLC.
The title compound was obtained pure as a white solid (50 mg).
Yield: 30%; (white powder); mp=109-110°C; [α]_{D}²⁰ = +91.9° (c=1.1, CHCl₃); LCMS (RT): 6.42 min (Method: E); MS (ES+) gave m/z: 371.2.
¹H-NMR (DMSO-d₆),δ (ppm): 8.28 (d, 1H); 8.04 (dd, 2H); 7.99 (ddd, 1H); 7.37 (dd, 2H); 7.20 (dd, 1H); 5.13 (m, 1H); 4.32 (m, 1H); 3.88 (dd, 1H); 3.70 (m, 1H); 3.50 (ddd, 1H); 2.46-2.25 (m, 2H); 1.93 (m, 1H); 1.76 (m, 1H).

### Example 22

### (4-Fluoro-2-methyl-phenyl)-[(S)-3-(5-phenyl-tetrazol-2-yl)-piperidin-1-yl]-methanone

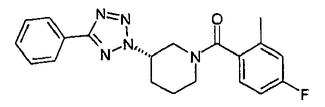

### 22(A) (4-Fluoro-2-methyl-phenyl)-((R)-3-hydroxy-piperidin-1-yl)-methanone

The title compound was prepared starting from (R)-3-hydroxy-piperidine hydrochloride and 2-methyl-4-fluorobenzoic acid, according to the same experimental procedure described in Example 1 (A).
Yield: 97%; LCMS (RT): 1.17 min (Method: B); MS (ES+) gave m/z: 238.3.

### 22(B) (4-Fluoro-2-methyl-phenyl)-[(S)-3-(5-phenyl-tetrazol-2-yl)-piperidin-1-yl]-methanone

The title compound was prepared following the same experimental procedure described in Example 5(B), starting from (4-fluoro-2-methyl-phenyl)-((R)-3-hydroxy-piperidin-1-yl)-methanone and phenyl-tetrazole.
Yield: 16%; (colourless gum); LCMS (RT): 7.00 min (Method: E); MS (ES+) gave m/z: 366.3.
¹H-NMR (DMSO-d₆), δ (ppm): 8.04 (m, 2H); 7.61-7.49 (m, 3H); 7.18 (dd, 1H); 7.05 (dd, 1H); 6.98 (ddd, 1H); 5.06 (m, 1H); 4.23 (m, 1H); 3.88 (dd, 1H); 3.57 (m, 1H); 3.39 (m, 1H); 2.46-2.28 (m, 2H); 2.21 (s, 3H); 1.94 (m, 1H); 1.71 (m, 1H).

### Example 23

### (4-Fluoro-2-methyl-phenyl)-{(S)-3-[5-(4-fluoro-phenyl)-tetrazol-2-yl]-piperidin-1-yl}-methanone

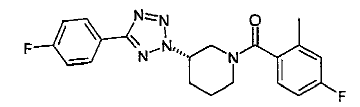

The title compound was prepared following the same experimental procedure described in Example 5(B), starting from (4-fluoro-2-methyl-phenyl)-((R)-3-hydroxy-piperidin-1-yl)-methanone and 4-fluorophenyl tetrazole.
Yield: 19% (colourless gum); LCMS (RT): 7.10 min (Method: E); MS (ES+) gave m/z: 384.3.
¹H-NMR (DMSO-d₆), δ (ppm): 8.09 (dd, 2H); 7.35 (dd, 2H); 7.18 (m, 1H); 7.05 (m, 1H); 6.99 (m, 1H); 5.05 (m, 1H); 4.23 (m, 1H); 3.88 (dd, 1H); 3.56 (m, 1H); 3.39 (m, 1H); 2.47-2.28 (m, 2H); 2.21 (s, 3H); 1.93 (m, 1H); 1.71 (m, 1H).

### Example 24

### (3,4-Difluoro-phenyl)- {(S)-3-[5-(4-fluoro-phenyl)-tetrazol-2-yl]-piperidin-1-yl}-methanone

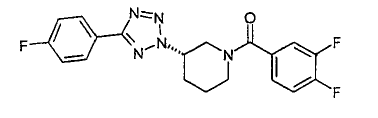

The title compound was prepared following the same experimental procedure described in Example 21, starting from (3,4-difluoro-phenyl)-((R)-3-hydroxy-piperidin-1-yl)-methanone (108 mg, 0.45 mmol) and 4-fluorophenyl tetrazole (150mg, 0.90 mmol).
The title compound was obtained pure by preparative HPLC, as a white solid (75 mg) Yield: 43%; (white solid); mp=123-128°C; [α]_{D}²⁰ = +70.2° (c=0.4, MeOH); LCMS (RT): 7.04 min (Method: E); MS (ES+) gave m/z: 388.2.
¹H-NMR (DMSO-d₆), δ (ppm): 8.08 (dd, 2H); 7.49-7.39 (m, 2H); 7.37 (dd, 2H); 7.24 (m, 1H); 5.11 (m, 1H); 4.28 (m, 1H); 3.85 (dd, 1H); 3.66 (m, 1H); 3.47 (m, 1H); 2.47-2.25 (m, 2H); 1.92 (m, 1H); 1.7 3(m, 1H).

### Example 25

### (2,4-Difluoro-phenyl)-{(S)-3-[5-(4-fluoro-phenyl)-tetrazol-2-yl]-piperidin-1-yl}-methanone

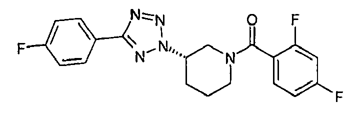

### 25(A) (2,4-Difluoro-phenyl)-((R)-3-hydroxy-piperidin-1-yl)-methanone

The title compound was prepared starting from (R)-3-hydroxy-piperidine hydrochloride and 2,4-difluorobenzoic acid, according to the same experimental procedure described in Example 1(A).
Yield: 86%; LCMS (RT): 2.5 min (Method: B); MS (ES+) gave m/z: 242.0.

### 25(B) (2,4-Difluoro-phenyl)- {(S)-3-[5-(4-fluoro-phenyl)-tetrazol-2-yl]-piperidin-1-yl} -methanone

The title compound was prepared following the same experimental procedure described in Example 21, starting from (2,4-difluoro-phenyl)-((R)-3-hydroxy-piperidin-1-yl)-methanone (108 mg, 0.45 mmol) and 4-fluorophenyl tetrazole (150 mg, 0.90 mmol).
The title compound was obtained pure by preparative HPLC, as a white solid (44 mg). Yield: 25%; (yellow solid); mp=65-68°C; [α]_{D}²⁰ = +54.1° (c=0.75, MeOH); LCMS (RT): 7.06 min (Method: E); MS (ES+) gave m/z: 388.2.
¹H-NMR (DMSO-d₆), δ (ppm): 8.08 (m, 2H); 7.46-7.33 (m, 3H); 7.22 (m, 1H); 7.11 (m 1H); 5.05 (m, 1H); 4.26 (m br, 1H); 3.88 (dd, 1H); 3.76-3.33 (m, 2H); 2.46-2.23 (m, 2H); 1.92 (m, 1H); 1.70 (m, 1H).

### Example 26

### (2,4-Difluoro-phenyl)-[(S)-3-(5-phenyl-tetrazol-2-yl)-piperidin-1-yl]-methanone

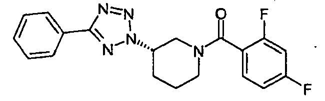

The title compound was prepared following the same experimental procedure described in Example 5(B), starting from (2,4-difluoro-phenyl)-((R)-3-hydroxy-piperidin-1-yl)-methanone and phenyltetrazole.
Yield: 16%; LCMS (RT): 6.93 min (Method: E); MS (ES+) gave m/z: 370.2.
¹H-NMR (CDCl₃), δ (ppm): 8.04 (m, 2H); 7.61-7.50 (m, 3H); 7.42 (m, 1H); 7.22 (m, 1H); 7.11 (m, 1H); 5.06 (m, 1H); 4.25 (m br, 1H); 3.88 (dd, 1H); 3.75-3.34 (m, 2H); 2.46-2.29 (m, 2H); 1.94 (m, 1H); 1.72 (m, 1H).

### Example 27 (Example DISCLAIMED)

### (4-Fluoro-phenyl)-[3-(5-phenyl-tetrazol-2-yl)-piperidin-1-yl]-methanone

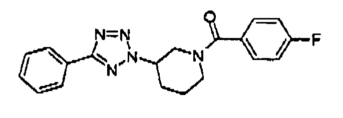

The title compound was prepared following the same experimental procedure described in Example 2(B), starting from (4-fluoro-phenyl)-3-hydroxy-piperidin-1-yl)-methanone and phenyltetrazole.
Yield: 42%; LCMS (RT): 7.04 min (Method A); MS (ES+) gave m/z: 352.1. ¹H-NMR (CDCl₃, 300 MHz, 323 K), δ (ppm): 8.12 (dd, 2H); 7.55-7.35 (m, 5H); 7.07 (dd, 2H); 4.90 (m, 1H); 4.50 (m, 1H); 4.33-3.69 (m, 3H); 3.36 (m, 1H); 2.57-2.28 (m, 1H); 2.14-1.96 (m, 1H); 1.84-1.66 (m,1H).

### Example 28

### (5-Methyl-isoxazol-4-yl)-[(S)-3-(5-phenyl-tetrazol-2-yl)-pipendin-1-yl]-methanone

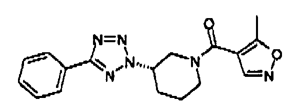

The title compound was prepared following the same experimental procedure described in Example 5(B), starting from ((R)-3-hydroxy-piperidin-1-yl)-(5-methyl-isoxazol-4-yl)-methanone and phenyl tetrazole.
Yield: 14%; (colourless gum); LCMS (RT); 6.24 min (Method: E); MS (ES+) gave m/z: 339.2.
¹H-NMR (DMSO-d₆, 373K), δ (ppm): 8.49(q, 1H); 8.04(m, 2H); 7.59-7.51(m, 3H); 5.08(m, 1H); 4.32(dd, 1H); 3.92(dd, 1H); 3.72(ddd, 1H); 3.54(ddd, 1H); 2.46-2.28(m, 2H); 2.44(s, 3H); 1.97(m, 1H); 1.76(m, 1H).

### PHARMACOLOGY:

The compounds provided in the present invention are positive allosteric modulators of mGluR5. As such, these compounds do not appear to bind to the orthosteric glutamate recognition site, and do not activate the mGluR5 by themselves. Instead, the response of mGluR5 to a concentration of glutamate or mGluR5 agonist is increased when compounds of Formula I are present. Compounds of Formula I are expected to have their effect at mGluR5 by virtue of their ability to enhance the function of the receptor.

### EXAMPLE A

### mGluR5 assay on rat cultured cortical astrocytes

Under exposure to growth factors (basic fibroblast growth factor, epidermal growth factor), rat cultured astrocytes express group I-Gq coupled mGluR transcripts, namely mGluR5, but none of the splice variants of mGluR1, and as a consequence, a functional expression of mGluR5 receptors (Miller et al. (1995) J. Neurosci. 15:6103-9): The stimulation of mGluR5 receptors with selective agonist CHPG and the full blockade of the glutamate-induced phosphoinositide (PI) hydrolysis and subsequent intracellular calcium mobilization with specific antagonist as MPEP confirm the unique expression of mGluR5 receptors in this preparation.
This preparation was established and used in order to assess the properties of the compounds of the present invention to increase the Ca²⁺ mobilization-induced by glutamate without showing any significant activity when applied in the absence of glutamate.

### Primary cortical astrocytes culture:

Primary glial cultures were prepared from cortices of Sprague-Dawley 16 to 19 days old embryos using a modification of methods described by Mc Carthy and de Vellis (1980) J. Cell Biol. 85:890-902 and Miller et al. (1995) J. Neurosci. 15(9):6103-9. The cortices were dissected and then dissociated by trituration in a sterile buffer containing 5.36 mM KCl, 0.44 mM NaHCO₃, 4.17 mM KH₂PO₄, 137 mM NaCl, 0.34 mM NaH₂PO₄, 1 g/L glucose. The resulting cell homogenate was plated onto poly-D-lysine precoated T175 flasks (BIOCOAT, Becton Dickinson Biosciences, Erembodegem, Belgium) in Dubelcco's Modified Eagle's Medium (D-MEM GlutaMAX^{™} I, Invitrogen, Basel, Switzerland) buffered with 25 mM HEPES and 22.7 mM NaHCO₃, and supplemented with 4.5g/L glucose, 1 mM pyruvate and 15 % fetal bovine serum (FBS, Invitrogen, Basel, Switzerland), penicillin and streptomycin and incubated at 37°C with 5% CO₂. For subsequent seeding, the FBS supplementation was reduced to 10 %. After 12 days, cells were subplated by trypsinisation onto poly-D-lysine precoated 384-well plates at a density of 20.000 cells per well in culture buffer.

### Ca²⁺ mobilization assay using rat cortical astrocytes:

After 1 day of incubation, cells were washed with Assay buffer containing: 142 mM NaCl. 6 mM KCl, 1 mM Mg₂SO₄, 1 mM CaCl₂, 20 mM HEPES, 1 g/L glucose, 0.125 mM sulfinpyrazone, pH 7.4. After 60 min of loading with 4 uM Fluo-4 (TefLabs, Austin, TX), the cells were washed three times with 50 µl of PBS Buffer and resuspended in 45 µl of Assay Buffer. The plates were then transferred to a Fluorometric Imaging Plate Reader (FLIPR, Molecular Devices, Sunnyvale, CA) for the assessment of intracellular calcium flux. After monitoring the baseline fluorescence for 10 s, a solution containing 10µM of representative compound of the present invention diluted in Assay Buffer (15 µl of 4X dilutions) was added to the cell plate in the absence or in the presence of 300 nM of glutamate. Under these experimental conditions, this concentration induces less than 20 % of the maximal response of glutamate and was the concentration used to detect the positive allosteric modulator properties of the compounds from the present invention. The final DMSO concentration in the assay was 0.3 %. In each experiment, fluorescence was then monitored as a function of time for 3 minutes and the data analyzed using Microsoft Excel and GraphPad Prism. Each data point was also measured two times.

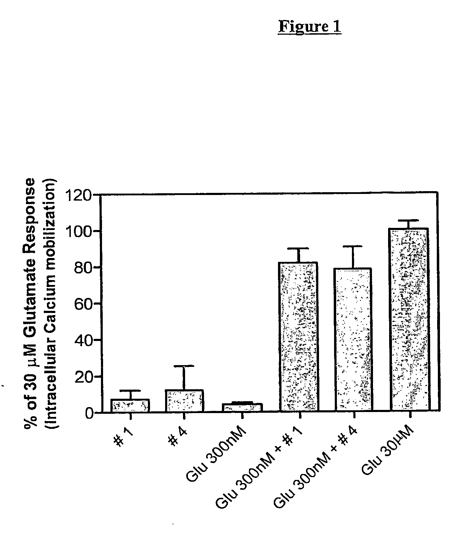

The results in Figure 1 represent the effect of 10 µM of example # 1 and #4 on primary cortical mGluR5-expressing cell cultures in the absence or in the presence of 300nM glutamate. Data are expressed as the percentage of maximal response observed with 30µM glutamate applied to the cells. Each bargraph is the mean and S.E.M of duplicate data points and is representative of three independent experiments

The results shown in Example A demonstrate that the compounds described in the present invention do not have an effect per se on mGluR5. Instead, when compounds are added together with an mGluR5 agonist such as glutamate, the effect measured is significantly potentiated compared to the effect of the agonist alone at the same concentration. This data indicates that the compounds of the present invention are positive allosteric modulators of mGluR5 receptors in native preparations.

Thus, the positive allosteric modulators provided in the present invention are expected to increase the effectiveness of glutamate or mGluR5 agonists at mGluR5 receptor. Therefore, these positive allosteric modulators are expected to be useful for treatment of various neurological and psychiatric disorders associated with glutamate dysfunction described to be treated herein and others that can be treated by such positive allosteric modulators.

### EXAMPLE B

### Animal Model of Schizophrenia

### Amphetamine model of schizophrenia

Amphetamine-induced increases in locomotor ambulation are well known and are widely used as a model of the positive symptoms of schizophrenia. This model is based on evidence that amphetamine increases motor behaviors and can induce a psychotic state in humans (Yui et al. (2000) Ann NY Acad Sci 914:1-12). Further, it is well known that amphetamine-induced increases in locomotor activity are blocked by antipsychotics drugs that are effective in the treatment of schizophrenia (Arnt (1995) Eur J Pharmacol 283:55-62). These results demonstrate that locomotor activation induced by amphetamine is a useful model for screening of compounds which may be useful in the treatment of schizophrenia.

**Subjects:** The present studies were performed in accordance with the animal care and use policies of Addex Pharmaceuticals and the laws and directives of France and the European Union governing the care and use of animals. Male C57BL6/j mice (20-30 g) 7 weeks of age at the time of delivery were group housed in a temperature and humidity controlled facility on a 12 hour light /dark cycle for at least 7 days before use. Mice had access to food and water ad libitum except during locomotor activity experiments.

**Assessment of locomotor (ambulatory) activity:** The effects of compounds on amphetamine-induced locomotor activation in mice were tested. Locomotor activity of mice was tested in white plastic boxes 35 cm X 35 cm square with walls 40 cm in height. Locomotor activity (ambulations) was monitored by a videotracking system (VideoTrack, Viewpoint, Champagne au Mont d'Or, France) that recorded the ambulatory movements of mice. Mice were naïve to the apparatus prior to testing. On test days, the test compound (30 mg/kg i.p. (intraperitoneal)) or vehicle were administered 30 minutes before the amphetamine sulphate (3.0 mg/kg s.c.). Mice were placed into the locomotor boxes immediately after the amphetamine injection and their locomotor activity, defined as the distance traveled in centimeters (cm), was measured for 60 minutes.

**Compound administration:** The compound was dissolved in a 5% DMSO/20% Tween 80/75% saline vehicle and administered in a volume of 10 ml/kg. Compound-vehicle-treated mice received the equivalent volume of vehicle solution i.p. in the absence of added compound. D-amphetamine sulfate (Amino AG, Neuenhof, Switzerland) was dissolved in saline and administered at a dose of 3.0 mg/kg s.c. in a volume of 10 ml/kg. D-amphetamine-vehicle-treated mice received an equivalent volume of saline vehicle injected s.c.

**Statistical analyses:** Statistical analyses were performed using GraphPad PRISM statistical software (GraphPad, San Diego, CA, USA). Data were analyzed using an unpaired t-test. The significance level was set at p<0.05.

### Effect of compounds on amphetamine-induced locomotor activity in mice

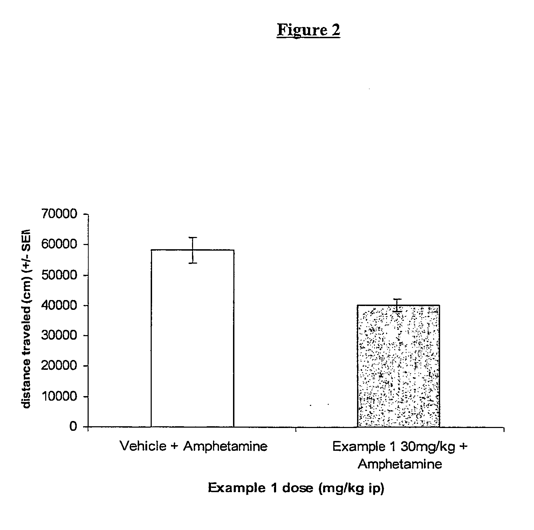

Data from such an experiment using a representative compound is shown in Figure 2.

Figure 2 shows that the representative compound of the invention at a dose of 30 mg/kg ip significantly attenuated the increase in locomotor activity induced by amphetamine (p < 0.01, t = 3.437, df = 13, vehicle-amphetamine group n = 8, example 1-amphetamine group n = 7).

### Summary of in vivo data

The data presented above show that the example #1 of the present invention significantly attenuate the hyperlocomotor effects of amphetamine, a widely accepted animal model of schizophrenia. These results support the potential of compounds of Formula I in the treatment of schizophrenia and related disorders.

The compounds of the present invention are allosteric modulators of mGluR5 receptor, they are useful for the production of medications, especially for the prevention or treatment of central nervous system disorders as well as other disorders modulated by this receptor.

The compounds of the invention can be administered either alone, or in combination with other pharmaceutical agents effective in the treatment of conditions mentioned above.

### FORMULATION EXAMPLES

Typical examples of recipes for the formulation of the invention are as follows:

### 1) Tablets

| | |
|---|---|
| Compound of the example 1 | 5 to 50 mg |
| Di-calcium phosphate | 20 mg |
| Lactose | 30 mg |
| Talcum | 10 mg |
| Magnesium stearate | 5 mg |
| Potato starch | ad 200 mg |

In this example, the compound of the example 1 can be replaced by the same amount of any of the described examples 1 to 26 and 28.

### 2) Suspension

An aqueous suspension is prepared for oral administration so that each 1 milliliter contains 1 to 5 mg of one of the described example, 50 mg of sodium carboxymethyl cellulose, 1 mg of sodium benzoate, 500 mg of sorbitol and water ad 1 ml.

### 3) Injectable

A parenteral composition is prepared by stirring 1.5 % by weight of active ingredient of the invention in 10% by volume propylene glycol and water.

### 4) Ointment

| | |
|---|---|
| Compound of the example 1 | 5 to 1000 mg |
| Stearyl alcohol | 3 g |
| Lanoline | 5 g |
| White petroleum | 15 g |
| Water | ad 100 g |

In this example, the compound I can be replaced by the same amount of any of the described examples 1 to 26 and 28.

## Claims

1. A compound having the formula I 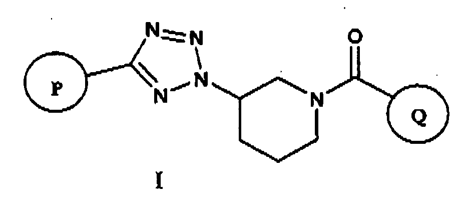 Wherein
P is an aryl or heteroaryl group of formula 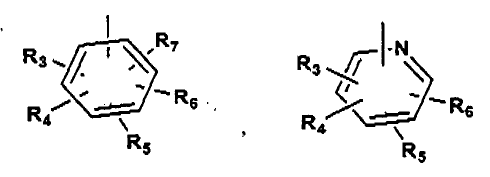
Q is an aryl or heteroaryl group of formula 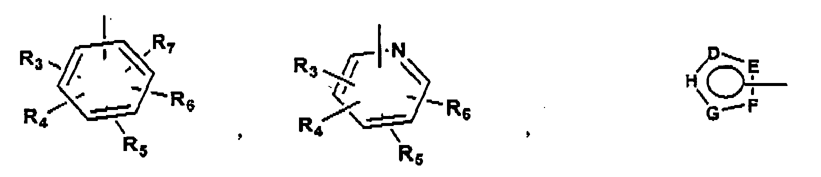
R₃, R₄, R₅, R₆, and R₇ independently are hydrogen, halogen, -(C₁- C₆)alkyl, -(C₃-C₆)cycloalkyl, -(C₃-C₇)cycloalkylalkyl, halo-(C₁-C₆)alkyl, -OR₈;
R₈ is hydrogen, (C₁-C₆)alkyl, (C₃-C₆)cycloalkyl, (C₃-C₇)cycloalkylalkyl, substituents;
D, E, F, G and H represent independently -C(R₃)=, -C(R₃)=C(R₄)-, -C(=O)-,-C(=S)-, -O-, -N=, -N(R₃)- or -S-;
Any N may be an N-oxide;
or a pharmaceutically acceptable salt, hydrate or solvate of such a compound; with the exclusion of the compound
(4-Fluoro-phenyl)-{3-[5-phenyl-tetrazol-2-yl]-piperidin-1-yl}-methanone

2. A compound according to claim 1, which can exist as optical isomers, wherein said compound is either the racemic mixture or an individual optical isomer.

3. A compound according to claim 1 or 2, wherein said compound is selected from:
(4-Fluoro-phenyl)-{(S)-3-[5-(4-fluoro-phenyl)-tetrazol-2-yl]-piperidin-1-yl}-methanone
(4-Fluoro-phenyl)-{3-[5-(4-fluoro-phenyl)-tetrazol-2-yl]-piperidin-l-yl}-methanone
(4-Fluoro-phenyl)-{3-[5-(2-fluoro-phenyl)-tetrazol-2-yl]-piperidin-l-yl}-methanone
(4-Fluoro-phenyl)-[(S)-3-(5-phenyl-tetrazol-2-yl)-piperidin-1-yl]-methanone
(4-Fluoro-phenyl)-[(R)-3-(5-phenyl-tetrazol-2-yl)-piperidin-1-yl]-methanone
{(S)-3-[5-(4-Chloro-phenyl)-tetrazol-2-yl]-piperidin-1-yl}-(4-fluoro-phenyl)-methanone
(4-Fluoro-phenyl)-{(S)-3-[5-(4-methoxy-phenyl)-tetrazol-2-yl]-piperidin-1-yl}-methanone
(4-Fluoro-phenyl)-{(S)-3-[5-(2-fluoro-phenyl)-tetrazol-2-yl]-piperidin-1-yl}-methanone
{(S)-3-[5-(2-Chloro-phenyl)-tetrazol-2-yl]-piperidin-1-yl}-(4-fluoro-phenyl)-methanone
(4-Fluoro-phenyl)-{(R)-3-[5-(4-fluoro-phenyl)-tetrazol-2-yl]-piperidin-1-yl}-methanone
(4-Fluoro-phenyl)-[(S)-3-(5-pyridin-4-yl-tetrazol-2-yl)-piperidin-1-yl]-methanone
(4-Fluoro-phenyl)-[(S)-3-(5-pyridin-3-yl-tetrazol-2-yl)-piperidin-1-yl]-methanone
(4-Fluoro-phenyl)-[(S)-3-(5-pyridin-2-yl-tetrazol-2-yl)-piperidin-1-yl]-methanone
(4-Fluoro-phenyl)-{(S)-3-[5-(2-methoxy-phenyl)-tetrazol-2-yl]-pipendin-1-yl}-methanone
(4-Fluoro-phenyl)-[(S)-3-(5-p-tolyl-tetrazol-2-yl)-piperidin-1-yl]-methanone
(4-Fluoro-phenyl)-[(S)-3-(5-m-tolyl-tetrazol-2-yl)-piperidin-1-yl]-methanone
(4-Fluoro-phenyl)-{{(S)-3-[5-(3-fluoro-phenyl)-tetrazol-2-yl]-piperidin-1-yl}-methanone
{(S)-3-[5-(4-Fluoro-phenyl)-tetrazol-2-yl]-piperidin-1yl}-(5-methyl-isoxazol-4-yl)-methanone
(6-Fluoro-pyridin-3-yl)-[(S)-3-(5-phenyl-tetrazol-2-yl)-piperidin-1-yl]-methanone
(3,4-Difluoro-phenyl)-[(S)-3-(5-phenyl-tetrazol-2-yl)-pipexidin-1-yl]-methanone
{(S)-3-[5-(4-Fluoro-phenyl)-tetrazol-2-yl]-piperidin-1-yl}-(6-fluoro-pyridin-3-yl)-methanone
(4-Fluoro-2-methyl-phenyl)-[(S)-3-(5-phenyl-tetrazol-2-yl)-piperidin-1-yl]-methanone
(4-Fluoro-2-methyl-phenyl)-{(S)-3-[5-(4-fluoro-phenyl)-tetrazol-2-yl]-piperidin-1-yl}-methanone
(3,4-Difluoro-phenyl)-{(S)-3-[5-(4-fluoro-phenyl)-tetrazol-2-yl]-piperidin-1-yl}-methanone
(2,4-Difluoro-phenyl)-{(S)-3-[5-(4-fluoro-phenyl)-tetrazol-2-yl]-piperidin-1-yl}-methanone
(2,4-Difluoro-phenyl)-[(S)-3-(5-phenyl-tetrazol-2-yl)-piperidin-1-yl]-methanone
(5-Methyl-isoxazol-4-yl)-[(S)-3-(5-phenyl-tetrazol-2-yl)-piperidin-1-yl]-methanone.

4. A pharmaceutical composition comprising a therapeutically effective amount of a compound according to claims 1 to 3 and a pharmaceutically acceptable carrier and/or excipient.

5. A compound/composition according to claims 1 to 4 for treating or preventing a condition in a mammal, including a human, the treatment or prevention of which is affected or facilitated by the neuromodulatory effect of mGluR5 allosteric modulators.

6. A compound/composition according to claims 1 to 4 for treating or preventing a condition in a mammal, including a human, the treatment or prevention of which is affected or facilitated by the neuromodulatory effect of mGluR5 positive allosteric modulators (enhancer).

7. A compound/composition according to claims 1 to 4 for treating or preventing central nervous system disorders selected from the group consisting of anxiety disorders: Agoraphobia, Generalized Anxiety Disorder (GAD), Obsessive-Compulsive Disorder (OCD), Panic Disorder, Posttraumatic Stress Disorder (PTSD), Social Phobia, Other Phobias, Substance-Induced Anxiety Disorder.

8. A compound/composition according to claims 1 to 4 for treating or preventing central nervous system disorders selected from the group consisting of childhood disorders: Attention-Deficit/Hyperactivity Disorder).

9. A compound/composition according to claims 1 to 4 for treating or preventing central nervous system disorders selected from the group consisting of eating Disorders (Anorexia Nervosa, Bulimia Nervosa).

10. A compound/composition according to claims 1 to 4 for treating or preventing central nervous system disorders selected from the group consisting of mood disorders: Bipolar Disorders (I & II), Cyclothymic Disorder, Depression, Dysthymic Disorder, Major Depressive Disorder, Substance-Induced Mood Disorder.

11. A compound/composition according to claims 1 to 4 for treating or preventing central nervous system disorders selected from the group consisting of psychotic disorders: Schizophrenia, Delusional Disorder, Schizoaffective Disorder, Schizophreniform Disorder, Substance-Induced Psychotic Disorder.

12. A compound/composition according to claims 1 to 4 for treating or preventing central nervous system disorders selected from the group consisting of cognitive disorders: Delirium, Substance-Induced Persisting Delirium, Dementia, Dementia Due to HIV Disease, Dementia Due to Huntington's Disease, Dementia Due to Parkinson's Disease, Dementia of the Alzheimer's Type, Substance-Induced Persisting Dementia, Mild Cognitive Impairment.

13. A compound/composition according to claims 1 to 4 for treating or preventing central nervous system disorders selected from the group consisting of personality disorders: Obsessive-Compulsive Personality Disorder, Schizoid, Schizotypal disorder.

14. A compound/composition according to claims 1 to 4 for treating or preventing central nervous system disorders selected from the group consisting of substance-related disorders: Alcohol abuse, Alcohol dependence, Alcohol withdrawal, Alcohol withdrawal delirium, Alcohol-induced psychotic disorder, Amphetamine dependence, Amphetamine withdrawal, Cocaine dependence, Cocaine withdrawal, Nicotine dependence, Nicotine withdrawal, Opioid dependence, Opioid withdrawal.

15. Use of a compound/composition according to claims 1 to 4 in the manufacture of a medicament for a treatment or prevention as defined in any of claims 7 to 14.

16. The use of a compound according to claim 1, 2 or 3 to prepare tracers for imaging metabotropic glutamate receptors.

## Patentansprüche

1. Verbindung der Formel I 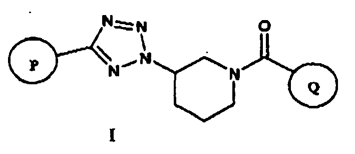 wobei
P eine Aryl- oder Heteroarylgruppe der folgenden Formel ist 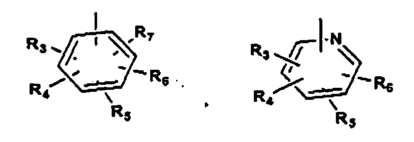
Q eine Aryl- oder Heteroarylgruppe der folgenden Formel ist 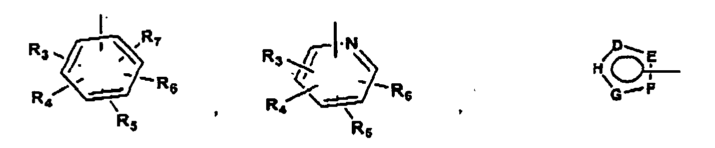
R₃, R₄, R₅, R₆ und R₇ unabhängig Wasserstoff, Halogen, -(C₁-C₆)Alkyl, (C₃-C₆)Cycloalkyl, -(C₃-C₇)Cycloalkylalkyl, Halo-(C₁-C₆)Alkyl, -OR₈ sind;
R₈ Wasserstoff, (C₁-C₆)Alkyl, (C₃-C₆)Cycloalkyl, (C₃-C₇)Cycloalkylalkyl, Substituenten ist;
D, E, F, G und H unabhängig -C(R₃)=, -C(R₃)=C(R₄)-, -C(=O)-, -C(=S)-, -O-, -N=, -N(R₃)- oder -S- repräsentieren;
jedes beliebige N ein N-Oxid sein kann;
oder ein pharmazeutisch akzeptables Salz, Hydrat oder Solvat einer solchen Verbindung; ausschließlich der Verbindung
(4-Fluor-phenyl)-{3-[5-phenyl-tetrazol-2-yl]-piperidin-1-yl}-methanon.

2. Verbindung nach Anspruch 1, die als optische Isomere vorliegen kann, wobei die genannte Verbindung entweder das racemische Gemisch oder ein individuelles optisches Isomer ist.

3. Verbindung nach Anspruch 1 oder 2, wobei die genannte Verbindung ausgewählt ist aus:
(4-Fluor-phenyl)-{(S)-3-[5-(4-fluor-phenyl)-tetrazol-2-yl]-piperidin-1-yl}-methanon
(4-Fluor-phenyl)-{3-[5-(4-fluor-phenyl)-tetrazol-2-yl]-piperidin-1-yl}-methanon
(4-Fluor-phenyl)-{3-[5-(2-fluor-phenyl)-tetrazol-2-yl]-piperidin-1-yl}-methanon
(4-Fluor-phenyl)-[(S)-3-(5-phenyl-tetrazol-2-yl)-piperidin-1-yl]-methanon
(4-Fluor-phenyl)-[(R)-3-(5-phenyl-tetrazol-2-yl)-piperidin-1-yl]-methanon
{(S)-3-[5-(4-Chlor-phenyl)-tetrazol-2-yl]-piperidin-1-yl}-(4-fluor-phenyl)-methanon
(4-Fluor-phenyl)-{(S)-3-[5-(4-methoxy-phenyl)-tetrazol-2-yl]-piperidin-1-yl}-methanon
(4-Fluor-phenyl)-{(S)-3-[5-(2-fluor-phenyl)-tetrazol-2-yl]-piperidin-1-yl}-methanon
{(S)-3-[5-(2-Chlor-phenyl)-tetrazol-2-yl]-piperidin-1-yl}-(4-fluor-phenyl)-methanon
(4-Fluor-phenyl)-{(R)-3-[5-(4-fluor-phenyl)-tetrazol-2-yl]-piperidin-1-yl}-methanon
(4-Fluor-phenyl)-[(S)-3-(5-pyridin-4-yl-tetrazol-2-yl)-piperidin-1-yl]-methanon
(4-Fluor-phenyl)-[(S)-3-(5-pyridin-3-yl-tetrazol-2-yl)-piperidin-1-yl]-methanon
(4-Fluor-phenyl)-[(S)-3-(5-pyridin-2-yl-tetrazol-2-yl)-piperidin-1-yl]-methanon
(4-Fluor-phenyl)-{(S)-3-[5-(2-methoxy-phenyl)-tetrazol-2-yl]-piperidin-1-yl}-methanon
(4-Fluor-phenyl)-[(S)-3-(5-p-tolyl-tetrazol-2-yl)-piperidin-1-yl]-methanon
(4-Fluor-phenyl)-[(S)-3-(5-m-tolyl-tetrazol-2-yl)-piperidin-1-yl]-methanon
(4-Fluor-phenyl)-{(S)-3-[5-(3-fluor-phenyl)-tetrazol-2-yl]-piperidin-1-yl}-methanon
{(S)-3-[5-(4-Fluor-phenyl)-tetrazol-2-yl]-piperidin-1-yl}-(5-methyl-isoxazol-4-yl)-methanon
(6-Fluor-pyridin-3-yl)-[(S)-3-(5-phenyl-tetrazol-2-yl)-piperidin-1-yl]-methanon
(3,4-Difluor-phenyl)-[(S)-3-(5-phenyl-tetrazol-2-yl)-piperidin-1-yl]-methanon
{(S)-3-[5-(4-Fluor-phenyl)-tetrazol-2-yl]-piperidin-1-yl}-(6-fluor-pyridin-3-yl)-methanon
(4-Fluor-2-methyl-phenyl)-[(S)-3-(5-phenyl-tetrazol-2-yl)-piperidin-1-yl]-methanon
(4-Fluor-2-methyl-phenyl)-{(S)-3-[5-(4-fluor-phenyl)-tetrazol-2-yl]-piperidin-1-yl}-methanon
(3,4-Difluor-phenyl)-{(S)-3-[5-(4-fluor-phenyl)-tetrazol-2-yl]-piperidin-1-yl}-methanon
(2,4-Difluor-phenyl)-{(S)-3-[5-(4-fluor-phenyl)-tetrazol-2-yl]-piperidin-1-yl}-methanon
(2,4-Difluor-phenyl)-[(S)-3-(5-phenyl-tetrazol-2-yl)-piperidin-1-yl]-methanon
(5-Methyl-isoxazol-4-yl)-[(S)-3-(5-phenyl-tetrazol-2-yl)-piperidin-1-yl]-methanon.

4. Pharmazeutische Zusammensetzung, die eine therapeutisch wirksame Menge einer Verbindung nach den Ansprüchen 1 bis 3 und einen pharmazeutisch akzeptablen Träger und/oder Exzipienten umfasst.

5. Verbindung/Zusammensetzung nach den Ansprüchen 1 bis 4 zur Behandlung oder Verhütung eines Zustands bei einem Säugetier, einschließlich eines Menschen, dessen Behandlung oder Verhütung durch den neuromodulatorischen Effekt von allosterischen mGluR5-Modulatoren beeinflusst oder erleichtert wird.

6. Verbindung/Zusammensetzung nach den Ansprüchen 1 bis 4 zur Behandlung oder Verhütung eines Zustands bei einem Säugetier, einschließlich eines Menschen, dessen Behandlung oder Verhütung durch den neuromodulatorischen Effekt von positiven allosterischen mGluR5-Modulatoren (Enhancer) beeinflusst oder erleichtert wird.

7. Verbindung/Zusammensetzung nach den Ansprüchen 1 bis 4 zur Behandlung oder Verhütung von Zentralnervensystemstörungen, ausgewählt aus der Gruppe bestehend aus Angststörungen: Agoraphobie, generalisierte Angststörung (GAD), obsessive Zwangsstörung (OCD), Panikstörung, posttraumatische Belastungsstörung (PTSD), soziale Phobie, andere Phobien, substanzinduzierte Angststörung.

8. Verbindung/Zusammensetzung nach den Ansprüchen 1 bis 4 zur Behandlung oder Verhütung von Zentralnervensystemstörungen, ausgewählt aus der Gruppe bestehend aus Kindheitsstörungen: Aufmerksamkeitsdefizit/Hyperaktivitätsstörung.

9. Verbindung/Zusammensetzung nach den Ansprüchen 1 bis 4 zur Behandlung oder Verhütung von Zentralnervensystemstörungen, ausgewählt aus der Gruppe bestehend aus Essstörungen (Magersucht, Ess-Brech-Sucht).

10. Verbindung/Zusammensetzung nach den Ansprüchen 1 bis 4 zur Behandlung oder Verhütung von Zentralnervensystemstörungen, ausgewählt aus der Gruppe bestehend aus Gemütszustandsstörungen: bipolare Störungen (I & II), zyklothyme Störung, Depressionen, dysthyme Störung, majore Depressionsstörung, substanzinduzierte Gemütszustandsstörung.

11. Verbindung/Zusammensetzung nach den Ansprüchen 1 bis 4 zur Behandlung oder Verhütung von Zentralnervensystemstörungen, ausgewählt aus der Gruppe bestehend aus psychotischen Störungen: Schizoprenie, Wahnstörung, schizoaffektive Störung, schizophreniforme Störung, substanzinduzierte psychotische Störung.

12. Verbindung/Zusammensetzung nach den Ansprüchen 1 bis 4 zur Behandlung oder Verhütung von Zentralnervensystemstörungen, ausgewählt aus der Gruppe bestehend aus kognitiven Störungen: Delirium, substanzinduziertes anhaltendes Delirium, Demenz, Demenz infolge der HIV-Krankheit, Demenz infolge der Huntington-Krankheit, Demenz infolge der Parkinson-Krankheit, Demenz vom Alzheimer-Typ, substanzinduzierte anhaltende Demenz, leichte kognitive Beeinträchtigung.

13. Verbindung/Zusammensetzung nach den Ansprüchen 1 bis 4 zur Behandlung oder Verhütung von Zentralnervensystemstörungen, ausgewählt aus der Gruppe bestehend aus Persönlichkeitsstörungen: Zwangspersönlichkeitsstörung, schizoide, schizotypische Störung.

14. Verbindung/Zusammensetzung nach den Ansprüchen 1 bis 4 zur Behandlung oder Verhütung von Zentralnervensystemstörungen, ausgewählt aus der Gruppe bestehend aus substanzbedingten Störungen: Alkoholmissbrauch, Alkoholabhängigkeit, Alkoholentzug, Alkoholentzugsdelirium, Alkohol-induzierte psychotische Störung, Amphetaminabhängigkeit, Amphetaminentzug, Kokainabhängigkeit, Kokainentzug, Nikotinabhängigkeit, Nikotinentzug, Opioidabhängigkeit, Opioidentzug.

15. Verwendung einer Verbindung/Zusammensetzung nach den Ansprüchen 1 bis 4 zur Herstellung eines Medikaments zur Behandlung oder Verhütung wie in einem der Ansprüche 7 bis 14 definiert.

16. Verwendung einer Verbindung nach Anspruch 1, 2 oder 3 zur Herstellung von Tracern zur Abbildung von metabotropen Glutamatrezeptoren.

## Revendications

1. Composé ayant la formule I 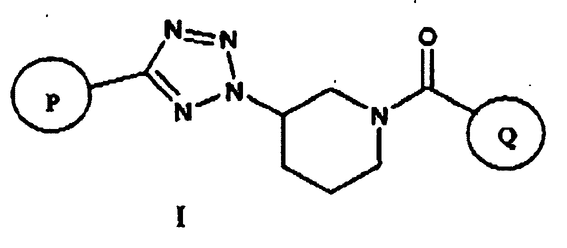 dans lequel
P est un groupe aryle ou hétéroaryle de la formule 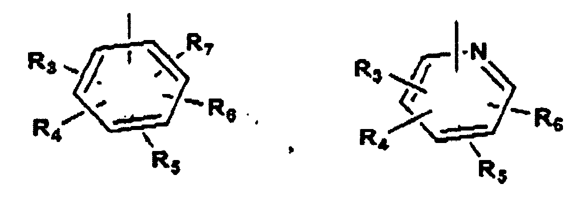
Q est un groupe aryle ou hétéroaryle de la formule 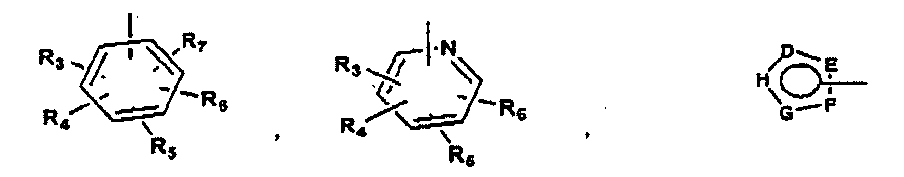
R₃, R₄, R₅, R₆, and R₇ sont indépendamment hydrogène, halogène, -(C₁-C₆)alkyle, -(C₃-C₆)cycloalkyle, -(C₃-C₇)cycloalkylalkyle, halo-(C₁C₆)alkyle, -OR₈;
R₈ est hydrogène, (C₁-C₆)alkyle, (C₃-C₆)cycloalkyle, (C₃-C₇)cycloalkylalkyle, des substituants ;
D, E, F, G et H représentent indépendamment -C(R₃)=, -C(R₃)=C(R₄)-, -C(=O)-,-C(=S)-,-O-N=, -N(R₃)- ou -S- ;
N'importe quel N peut être un N-oxyde ;
ou bien un sel pharmaceutiquement acceptable, un hydrate ou solvat d'un composé de ce type ; à l'exclusion du composé
(4-Fluoro-phényl)-{3-[5-phényi-tétrazol-2-yl]-pipéridine-1-yl}-méthanone

2. Composé selon la revendication 1, qui peut exister en tant qu'isomères optiques, dans lequel ledit composé est soit le mélange racémique soit un isomère optique individuel.

3. Composé selon la revendication 1 ou 2, dans lequel ledit composé est sélectionné parmi :
(4-Fluoro-phényl)-{(S)-3-[5-(4-fluoro-phényl)-tétrazol-2-yl]-pipéridine-1-yl}-méthanone
(4-Fluoro-phényl)-{3-[5-(4-fluoro-phényl)-tétrazol-2-yl]-pipéridine-1-yl}-méthanone
(4-Fluoro-phényl)-{3-[5-(2-fluoro-phényl)-tétrazol-2-yl]-pipéridine-1-yl)-méthanone
(4-Fluoro-phényl)-t(S)-3-(5-phényl-tétrazol-2-yl)-pipéridine-1-yl]-méthanone
(4-Fluoro-phényl)-[(R)-3-(5-phényl-tétrazol-2-yl)-pipéridine-1-yl]-méthanone
{(S)-3-[5-(4-Chloro-phényl)-tétrazol-2-yl]-pipéridine-1-yl}-(4-fluoro-phényl)-méthanone
(4-Fluoro-phényl)-{(S)-3-[5-(4-méthoxy-phényl)-tétrazol-2-yl]-pipéridine-1-yl}-méthanone
(4-Fluoro-phény))-{(S)-3-[5-(2-fluoro-phényl)-tétrazol-2-yl]-pipéridine-1-yl}-méthanone
{(S)-3-[5-(2-Chloro-phényl)-tétrazol-2-yl]-pipéridine-1-yl}-(4-fluoro-phény>méthanone
(4-Fluoro-phényl)-{(R)-3-[5-(4-fluoro-phényl)-tétrazol-2-yl]-pipéridine-1-yl}-méthanone
(4-Fluoro-phényl)-[(S)-3-(5-pyridine-4-yl-tétrazol-2-yl)-pipéridine-1-yl]-méthanone
(4-Fluoro-phényl)-[(S)-3-(5-pyridine-3-yl-tétrazol-2-yl)-pipéridine-1-yl]-méthanone
(4-Fluoro-phényl)-[(S)-3-(5-pyridine-2-yl-tétrazol-2-yl)-pipéridine-1-yl]-méthanone
(4-Fluoro-phényl)-{(S)-3-[5-(2-méthoxy-phényl)-tétrazol-2-yl]-pipéridine-1-yl}-méthanone
(4-Fluoro-phényl)-[(S)-3-(5-p-tolyl-tétrazol-2-yl)-pipéridine-1-yl]-méthanone
(4-Fluoro-phényl)-[(5)-3-(5-m-tolyl-tétrazol-2-yl)-pipéridine-1-yl]-méthanone
(4-Fluoro-phényl)-{(S)-3-[5-(3-fluoro-phényl)-tétrazol-2-yl]-pipéridine-1-yl}-méthanone
{(S)-3-[5-(4-Fluoro-phényl)-tétrazol-2-yl]-pipéridine-1-yl}-(5-méthyl-isoxazol-4-yl)-méthanone
(6-Fluoro-pyridine-3-yl)-[(S)-3-(5-phényl-tétrazol-2-yl)-pipéridine-1-yl]-méthanone
(3,4-Difluoro-phényl)-[(S)-3-(5-phényl-tétrazol-2-yl)-pipéridine-1-yl]-méthanone
{(S)-3-[5-(4-Fluoro-phényl)-tétrazol-2-yl]-pipéridine-1-yl}-(6-fluoro-pyridine-3-yl)-méthanone
(4-Fluoro-2-méthyl-phényl)-[(S)-3-(5-phényl-tétrazol-2-yl)-pipéridine-1-yl]-méthanone
(4-Fluoro-2-méthyl-phényl)-{(S)-3-[5-(4-fluoro-phényl)-tétrazol-2-yl]-pipéridine-1-yl}-méthanone
(3,4-Difluoro-phényl)-{(S)-3-[5-(4-fluoro-phényl)-tétrazol-2-yl]-pipéridine-1-yl}-méthanone
(2,4-Difluoro-phényl)-{(S)-3-[S-(4-fluoro-phényl)-tétrazol-2-yl]-pipéridine-1-yl}-méthanone
(2,4-Difluoro-phényl)-[(S)-3-(5-phényl-tétrazol-2-yl)-pipéridine-1-yl]-méthanone
(5-Méthyl-isoxazol-4-yl)-[(S)-3-(5-phényl-tétrazol-2-yl)-pipéridine-1-yl]-méthanone.

4. Composition pharmaceutique comprenant une quantité thérapeutiquement efficace d'un composé selon les revendications 1 à 3 ainsi qu'un vecteur et/ou excipient pharmaceutiquement acceptable.

5. Composé/composition selon les revendications 1 à 4, pour le traitement ou la prévention d'une affection chez un mammifère, y compris un être humain, dont le traitement ou la prévention est affecté ou facilité par l'effet neuromodulateur des modulateurs allostériques des mGluR5.

6. Composé/composition selon les revendications 1 à 4, pour le traitement ou la prévention d'une affection chez un mammifère, y compris un être humain, dont le traitement ou la prévention est affecté ou facilité par l'effet neuromodulateur des modulateurs allostériques positifs des mGluR5 (activateur).

7. Composé/composition selon les revendications 1 à 4, pour le traitement ou la prévention de troubles du système nerveux central sélectionnés parmi le groupe comprenant des troubles d'anxiété : agoraphobie, trouble d'anxiété généralisée, trouble obsessivo-compulsif, trouble panique, trouble de stress post-traumatique, phobie sociale, autres phobies, trouble d'anxiété dû aux substances psycho-actives.

8. Composé/composition selon les revendications 1 à 4, pour le traitement ou la prévention de troubles du système nerveux central sélectionnés parmi le groupe comprenant des troubles de l'enfance : trouble d'hyperactivité avec déficit de l'attention.

9. Composé/composition selon les revendications 1 à 4, pour le traitement ou la prévention de troubles du système nerveux central sélectionnés parmi le groupe comprenant des troubles de l'alimentation (anorexie mentale, boulimie mentale).

10. Composé/composition selon les revendications 1 à 4, pour le traitement ou la prévention de troubles du système nerveux central sélectionnés parmi le groupe comprenant des troubles de l'humeur : troubles bipolaires (I et II), trouble cyclothymique, dépression, trouble dysthymique, trouble dépressif majeur, trouble de l'humeur dû aux substances psycho-actives.

11. Composé/composition selon les revendications 1 à 4, pour le traitement ou la prévention de troubles du système nerveux central sélectionnés parmi le groupe comprenant des troubles psychotiques : schizophrénie, trouble délirant, trouble schizo-affectif, trouble schizophréniforme, trouble psychotique dû aux substances psycho-actives.

12. Composé/composition selon les revendications 1 à 4, pour le traitement ou la prévention de troubles du système nerveux central sélectionnés parmi le groupe comprenant des troubles cognitifs : délire, délire persistant dû aux substances psycho-actives, démence, démence due au VIH, démence due à la maladie de Huntington, démence due à la maladie de Parkinson, démence de type Alzheimer, démence persistante due aux substances psycho-actives, trouble cognitif léger.

13. Composé/composition selon les revendications 1 à 4, pour le traitement ou la prévention de troubles du système nerveux central sélectionnés parmi le groupe comprenant des troubles de la personnalité : trouble de la personnalité obsessivo-compulsif, trouble schizoïde, trouble schizotypique.

14. Composé/composition selon les revendications 1 à 4, pour le traitement ou la prévention de troubles du système nerveux central sélectionnés parmi le groupe comprenant des troubles dus aux substances psycho-actives : abus d'alcool, dépendance à l'alcool, sevrage de l'alcool, délire du sevrage de l'alcool, trouble psychotique dû à l'alcool, dépendance aux amphétamines, sevrage des amphétamines, dépendance à la cocaïne, sevrage de la cocaïne, dépendance à la nicotine, sevrage de la nicotine, dépendance aux opiacés, sevrage des opiacés.

15. Utilisation d'un composé/d'une composition selon les revendications 1 à 4 dans la fabrication d'un médicament pour un traitement ou une prévention tels que définis dans l'une quelconque des revendications 7 à 14.

16. Utilisation d'un composé selon la revendication 1, 2 ou 3 pour préparer des traceurs servant à la visualisation de récepteurs métabotropiques du glutamate.
